(19) Europäisches Patentamt
European Patent Office
Office européen des brevets

(11) Veröffentlichungsnummer: **0 465 970 A1**

# (12) EUROPÄISCHE PATENTANMELDUNG

(21) Anmeldenummer: **91110832.2**

(22) Anmeldetag: **29.06.91**

(51) Int. Cl.[5]: **C07D 471/04**, C07F 9/6561, C07F 9/32, A61K 31/435, //(C07D471/04,221:00,209:00)

(30) Priorität: **13.07.90 DE 4022414**

(43) Veröffentlichungstag der Anmeldung: **15.01.92 Patentblatt 92/03**

(84) Benannte Vertragsstaaten: **AT BE CH DE DK ES FR GB GR IT LI LU NL SE**

(71) Anmelder: **BAYER AG**

**W-5090 Leverkusen 1 Bayerwerk(DE)**

(72) Erfinder: **Hübsch, Walter, Dr.**
**Am Eckbusch 43/50**
**W-5600 Wuppertal 1(DE)**
Erfinder: **Angerbauer, Rolf, Dr.**
**Moospfad 20**
**W-5600 Wuppertal 1(DE)**
Erfinder: **Fey, Peter, Dr.**
**Ursulastrasse 14**
**W-5600 Wuppertal 1(DE)**
Erfinder: **Bischoff, Hilmar, Dr.**
**Am Rohm 78**
**W-5600 Wuppertal 1(DE)**
Erfinder: **Bender, Joachim, Dr.**
**Donnenberger Strasse 68**
**W-5620 Velbert 15(DE)**
Erfinder: **Schmidt, Delf, Dr.**
**Am Eckbusch 55b**
**W-5600 Wuppertal 1(DE)**

## (54) Substituierte Pyrrolopyridine.

(57) Substituierte Pyrrolo-pyridine können durch Reduktion der entsprechenden Ketone hergestellt werden. Sie sind wertvolle Wirkstoffe für Arzneimittel und können beispielsweise zur Behandlung von Hyperlipoproteinämie Lipoproteinämie und Arteriosklerose eingesetzt werden.

EP 0 465 970 A1

Die Erfindung betrifft substituierte Pyrrolo-pyridine, Zwischenverbindungen zu ihrer Herstellung, ihre Herstellung und ihre Verwendung in Arzneimitteln.

Es ist bekannt, daß aus Pilzkulturen isolierte Lactonderivate Inhibitoren der 3-Hydroxy-3-methyl-glutaryl Coenzym A Reduktase (HMG-CoA-Reduktase) sind [Mevinolin, EP 22 478; US 4 231 938].

Außerdem werden in der DE 38 17 298 A1 phosphorhaltige HMG-CoA-Reduktase-Inhibitoren mit antihypercholesterolemischer Aktivität publiziert.

Es wurden neue substituierte Pyrrolo-pyridine der allgemeinen Formel (I)

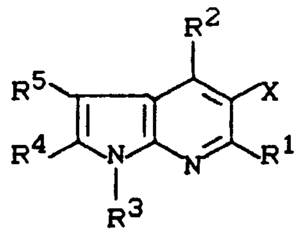

(I)

in welcher

R[1] - für geradkettiges oder verzweigtes Alkyl mit bis zu 8 Kohlenstoffatomen steht,
- für Cycloalkyl mit 3 bis 6 Kohlenstoffatomen steht,

R[2] - für Phenyl steht, das gegebenenfalls bis zu 2-fach gleich oder verschieden durch geradkettiges oder verzweigtes Alkyl mit bis zu 6 Kohlenstoffatomen, Trifluormethyl, Hydroxymethyl, Phenoxy, Benzyl, Benzyloxy oder Halogen substituiert ist,

R[3] - für Wasserstoff oder
- für geradkettiges oder verzweigtes Alkyl mit bis zu 10 Kohlenstoffatomen steht, das gegebenenfalls durch Cyano, geradkettiges oder verzweigtes Alkoxy mit bis zu 6 Kohlenstoffatomen, Halogen, Pyridyl, Chinolinyl, Furyl, Thienyl, Naphthyl oder Phenyl substituiert ist, das seinerseits durch Halogen, Cyano, Nitro, geradkettiges oder verzweigtes Alkyl oder Alkoxy mit jeweils bis zu 6 Kohlenstoffatomen substituiert sein kann,
- für geradkettiges oder verzweigtes Alkenyl oder Alkinyl mit jeweils bis zu 8 Kohlenstoffatomen steht, oder
- für Cycloalkyl mit 3 bis 6 Kohlenstoffatomen oder Phenyl steht,

R[4] und R[5] gleich oder verschieden sind und
- für Wasserstoff oder
- für geradkettiges oder verzweigtes Alkyl mit bis zu 6 Kohlenstoffatomen stehen, das gegebenenfalls durch Hydroxy, Halogen, Cyano oder Alkoxy mit bis zu 4 Kohlenstoffatomen substituiert ist,

X - für einen Rest der Formel -A-B steht,
worin

A - eine Gruppe der Formel $-CH_2-CH_2-$ oder $-CH=CH-$bedeutet,

B - eine Gruppe der Formel

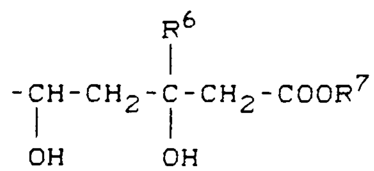

oder

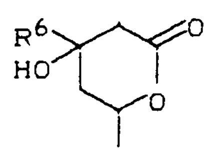

bedeutet
worin

R[6] - Wasserstoff oder geradkettiges oder verzweigtes Alkyl mit bis zu 10 Kohlenstoffatomen bedeutet
und

R[7] - Wasserstoff oder geradkettiges oder verzweigtes Alkyl mit bis zu 10 Kohlenstoffatomen bedeutet, das durch Phenyl substituiert sein kann, oder
- Aryl mit 6 bis 10 Kohlenstoffatomen oder ein Kation bedeutet,
oder

- für einen Rest der Formel

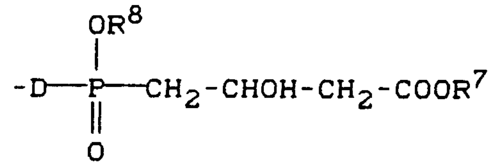

steht,
worin

D - einen Rest der Formel $-(CH_2)t$, $-CH=CH-$, $-C \equiv C-$ oder $-CH_2-O-$ bedeutet, worin letzterer über O an das Phosphoratom gebunden ist,

$R^7$ die oben angegebene Bedeutung von $R^7$ hat,

$R^8$ - Wasserstoff oder geradkettiges oder verzweigtes Alkyl mit bis zu 4 Kohlenstoffatomen bedeutet,

t - die Zahl 1 oder 2 bedeutet,

und deren Salze gefunden.

Bildet $R^7$ mit der Carboxygruppe einen Esterrest so ist hiermit bevorzugt ein physiologisch verträglicher Esterrest gemeint, der in vivo leicht zu einer freien Carboxylgruppe und einem entsprechenden physiologisch verträglichen Alkohol hydrolysiert wird. Hierzu gehören beispielsweise Alkylester ($C_1$ bis $C_6$) und Aralkylester ($C_7$ bis $C_{10}$), bevorzugt ($C_1$-$C_4$)-Alkylester sowie Benzylester. Darüberhinaus seien die folgenden Esterreste genannt: Methylester, Ethylester, Propylester, Benzylester.

Steht $R^7$ für ein Kation, so ist bevorzugt ein physiologisch verträgliches Metall- oder Ammoniumkation gemeint. Bevorzugt sind hierbei Alkali-bzw. Erdalkalikationen wie beispielsweise Natrium-, Kalium-, Magnesium- oder Calciumkationen, sowie Aluminium- oder Ammoniumkationen, sowie nichttoxische substituierte Ammoniumkationen aus Aminen wie ($C_1$-$C_4$)-Dialkylamine, ($C_1$-$C_4$)-Trialkylamine, Prokain, Dibenzylamin, N,N'-Dibenzylethylendiamin, N-Benzyl-$\beta$-phenylethylamin, N-Methylmorpholin oder N-Ethylmorpholin, 1-Ephenamin, Dihydroabietylamin, N,N'-Bis-dihydroabietylethylendiamin, N-Niederalkylpiperidin und andere Amine, die zur Bildung von Salzen verwendet werden können.

Ebenso kann $R^8$ für eines der oben aufgeführten physiologisch verträglichen Metall- oder Ammoniumkationen stehen.

Überraschenderweise zeigen die erfindungsgemäßen substituierten Pyrrolo-pyridine eine überlegene inhibitorische Wirkung auf die HMG-CoA-Reduktase (3-Hydroxy-3-methyl-glutaryl-Coenzym A Reduktase).

Bevorzugt sind Verbindungen der allgemeinen Formel (I),
in welcher

$R^1$ - für geradkettiges oder verzweigtes Alkyl mit bis zu 6 Kohlenstoffatomen steht,
- für Cyclopropyl, Cyclopentyl oder Cyclohexyl steht,

$R^2$ - für Phenyl steht, das gegebenenfalls durch geradkettiges oder versweigtes Alkyl mit bis zu 4 Kohlenstoffatomen, Trifluormethyl, Fluor, Chlor oder Brom substituiert ist,

$R^3$ - für Wasserstoff oder
- für geradkettiges oder verzweigtes Alkyl mit bis zu 8 Kohlenstoffatomen steht, das gegebenenfalls durch Cyano, geradkettiges oder verzweigtes Alkoxy mit bis zu 4 Kohlenstoffatomen, Fluor, Chlor, Brom, Pyridyl oder Phenyl substituiert ist, das seinerseits durch Fluor, Chlor, Brom, Cyano, Nitro, geradkettiges oder verzweigtes Alkyl oder Alkoxy mit jeweils bis zu 4 Kohlenstoffatomen substituiert sein kann,
- für geradkettiges oder verzweigtes Alkenyl oder Alkinyl mit jeweils bis zu 6 Kohlenstoffatomen steht,
- für Cyclopropyl, Cyclopentyl, Cyclohexyl oder Phenyl steht,

$R^4$ und $R^5$ für Wasserstoff stehen,

X - für einen Rest der Formel -A-B steht,
worin

A - eine Gruppe der Formel $-CH_2-CH_2-$ oder $-CH=CH-$bedeutet,

B - eine Gruppe der Formel

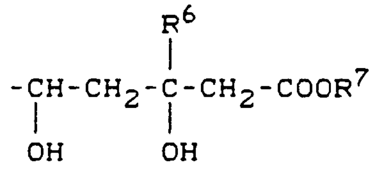 oder 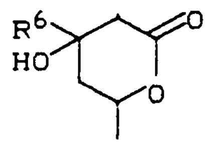

bedeutet,
worin

$R^6$ - Wasserstoff bedeutet
und

$R^7$ - Wasserstoff oder geradkettiges oder verzweigtes Alkyl mit bis zu 8 Kohlenstoffatomen oder Benzyl bedeutet, oder
- Phenyl oder ein Kation bedeutet

und deren Salze.

Besonders bevorzugt sind Verbindungen der allgemeinen Formel (I),
in welcher

$R^1$ - für geradkettiges oder verzweigtes Alkyl mit bis zu 4 Kohlenstoffatomen oder Cyclopropyl steht,

$R^2$ - für Phenyl steht, das gegebenenfalls durch Methyl, Trifluormethyl, Fluor oder Chlor substituiert ist,

$R^3$ - für Wasserstoff oder
- für geradkettiges oder verzweigtes Alkyl mit bis zu 6 Kohlenstoffatomen steht, das gegebenenfalls durch Cyano, Methoxy, Fluor, Pyridyl oder Phenyl substituiert ist,
- für geradkettiges oder verzweigtes Alkenyl oder Alkinyl mit jeweils bis zu 4 Kohlenstoffatomen steht,
- für Cyclopentyl oder Cyclohexyl steht,

$R^4$ und $R^5$ für Wasserstoff stehen,

X - für einen Rest der Formel -A-B steht,
worin

A - eine Gruppe $-CH_2-CH_2-$ oder $-CH=CH-$ bedeutet,

B - eine Gruppe der Formel

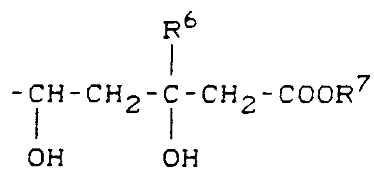 oder 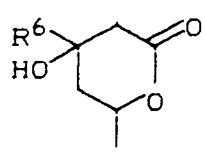

bedeutet,
worin

$R^6$ - Wasserstoff bedeutet
und

$R^7$ - Wasserstoff, Methyl, Ethyl, Propyl, Isopropyl, Butyl, Isobutyl, tert.Butyl oder Benzyl bedeutet, oder
- ein Natrium-, Kalium-, Calcium-, Magnesium- oder Ammoniumion bedeutet

und deren Salze.

Die erfindungsgemäßen substituierten Pyrrolo-pyridine der allgemeinen Formel (I) haben mehrere asymmetrische Kohlenstoffatome und können daher in verschiedenen stereochemischen Formen existieren. Sowohl die einzelnen Isomeren als auch deren Mischungen sind Gegenstand der Erfindung.

Je nach der Bedeutung der unter dem Substituenten X aufgeführten Reste ergeben sich unterschiedliche Stereoisomere, die im folgenden am Beispiel des Restes -A-B erläutert werden sollen:

a) Steht die Gruppe -A- für eine Gruppe der Formel $-CH=CH-$, so können die erfindungsgemäßen Verbindungen in zwei stereoisomeren Formen existieren, die an der Doppelbindung E-konfiguriert (II) oder Z-konfiguriert (III) sein können:

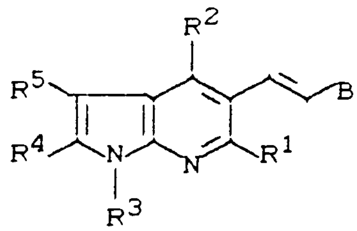

(II) E-Form

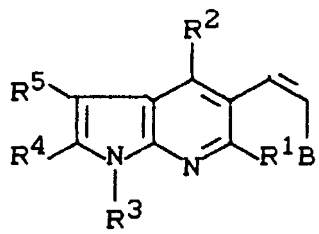

(III) Z-Form

in welcher
$R^1$, $R^2$, $R^3$, $R^4$, $R^5$ und B die oben angegebene Bedeutung haben.

Bevorzugt sind diejenigen Verbindungen der allgemeinen Formel (I) die E-konfiguriert sind (II).

b) Steht der Rest -B für eine Gruppe der Formel

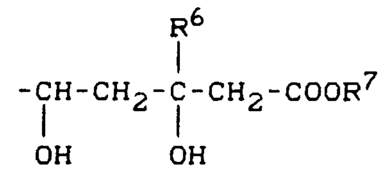

so besitzen die Verbindungen der allgemeinen Formel (I) mindestens zwei asymmetrische Kohlenstoffatome, nämlich die beiden Kohlenstoffatome an denen die Hydroxygruppen gebunden sind. Je nach der relativen Stellung dieser Hydroxygruppen zueinander, können die erfindungsgemäßen Verbindungen in der erythro-Konfiguration (IV) oder in der threo-Konfiguration (V) vorliegen.

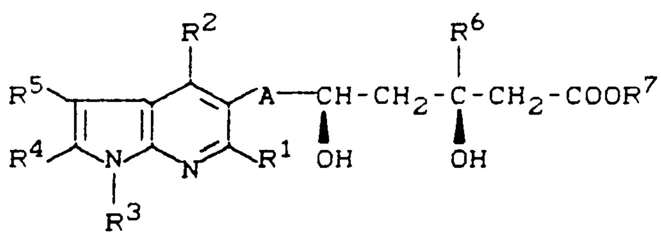

Erythro-Form (IV)

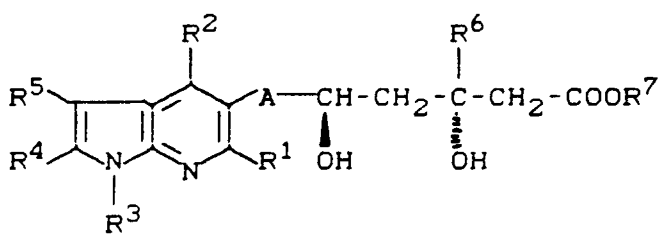

Threo-Form (V)

Sowohl von den Verbindungen in Erythro- als auch in Threo-Konfiguration existieren wiederum jeweils zwei Enantiomere, nämlich 3R,5S-Isomeres bzw. 3S,5R-Isomeres (Erythro-Form) sowie 3R,5R-Isomeres und 3S,5S-Isomeres (Threo-Form).

Bevorzugt sind hierbei die Erythro-konfigurierten Isomeren, besonders bevorzugt das 3R,5S-Isomere

sowie das 3R,5S-3S,5R-Racemat.

c) Steht der Rest -B für eine Gruppe der Formel

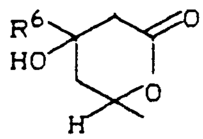

so besitzen die substituierten Pyrrolo-pyridine mindestens zwei asymmetrische Kohlenstoffatome, nämlich das Kohlenstoffatom an dem die Hydroxygruppe gebunden ist, und das Kohlenstoffatom an welchem der Rest der Formel

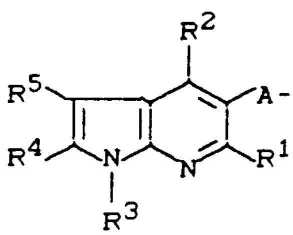

gebunden ist. Je nach der Stellung der Hydroxygruppe zur freien Valenz am Lactonring können die substituierten Pyrrolo-pyridine als cis-Lactone (VI) oder als trans-Lactone (VII) vorliegen.

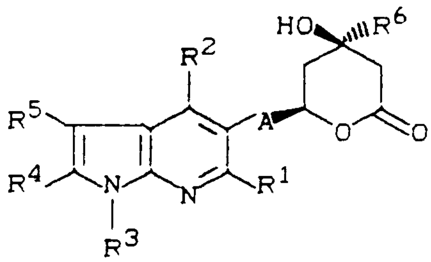

cis-Lacton (VI)

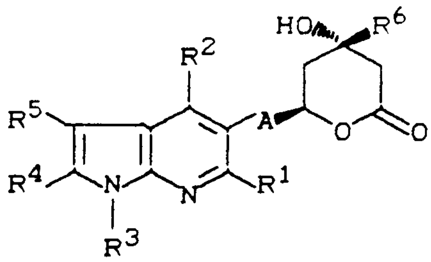

trans-Lacton (VII)

Sowohl vom cis-Lacton aus als auch vom trans-Lacton existieren wiederum jeweils zwei Isomere nämlich das 4R,6R-Isomere bzw. das 4S,6S-Isomere (cis-Lacton), sowie das 4R,6S-Isomere bzw. 4S,6R-Isomere (trans-Lacton). Bevorzugte Isomere sind die trans-Lactone. Besonders bevorzugt ist hierbei das 4R,6S-Isomere (trans) sowie das 4R,6S-4S,6R-Racemat.

Beispielsweise seien die folgenden isomeren Formen der substituierten Pyrrolo-pyridine genannt:

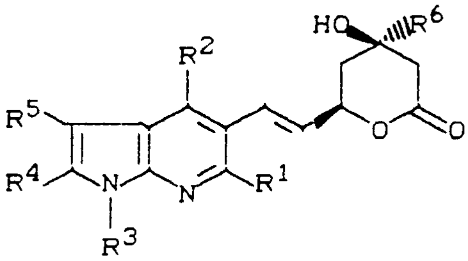

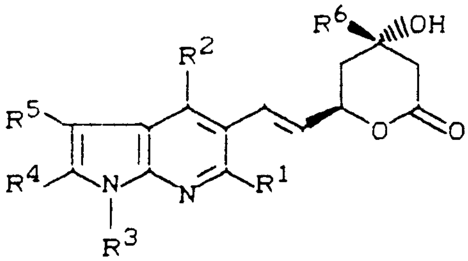

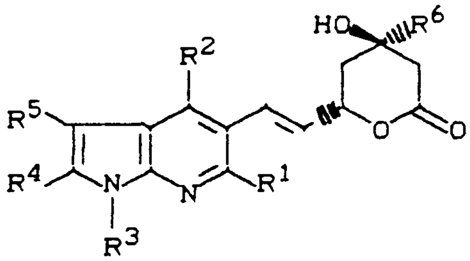

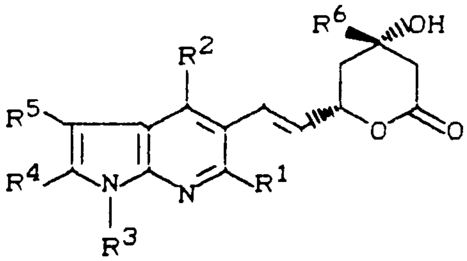

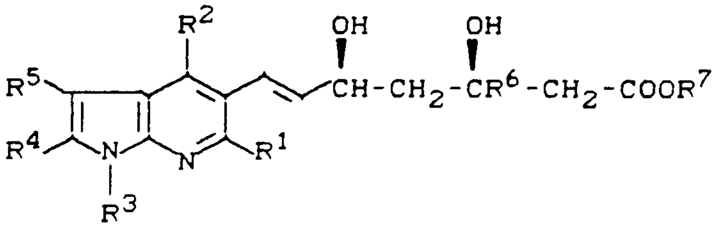

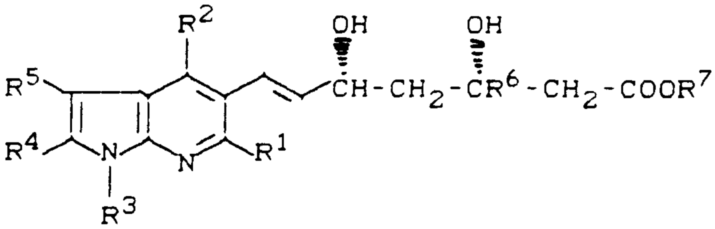

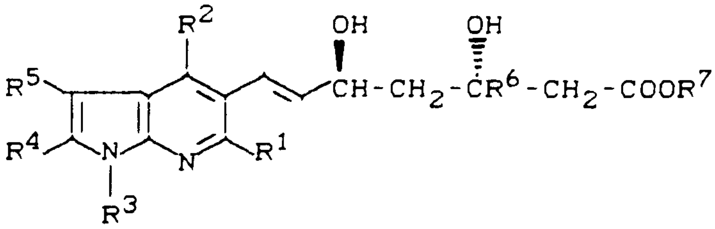

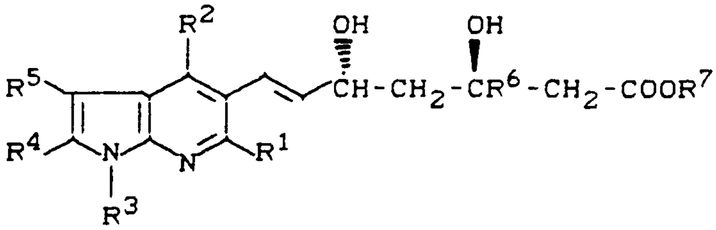

Die Herstellung der substiuierten Pyrrolo-pyridine der allgemeinen Formel (I) erfolgt, indem man Ketone der allgemeinen Formel (VIII)

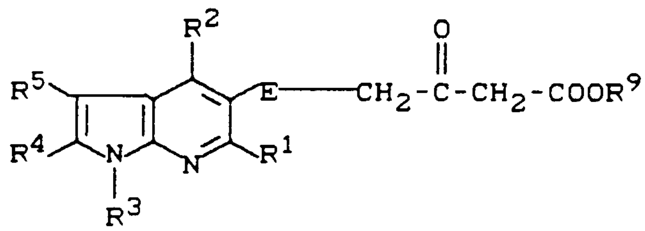

(VIII)

in welcher

| | |
|---|---|
| $R^1$, $R^2$, $R^3$, $R^4$ und $R^5$ | die oben angegebene Bedeutung haben, |
| $R^9$ | - für Alkyl steht, |
| | und |
| E | für eine Gruppe |

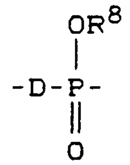

(VIIIa)

oder

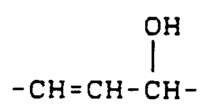

(VIIIb)

steht
reduziert,
im Fall der Herstellung der Säuren die Ester verseift,
im Fall der Herstellung der Lactone die Carbonsäuren cyclisiert,
im Fall der Herstellung der Ethylenverbindungen (A = $-CH_2-CH_2-$) die Ethenylverbindungen (A = -CH = CH-) nach üblichen Methoden hydriert,
und gegebenenfalls Isomeren trennt.

Das erfindungsgemäße Verfahren kann durch das folgende Formelschema erläutert werden:

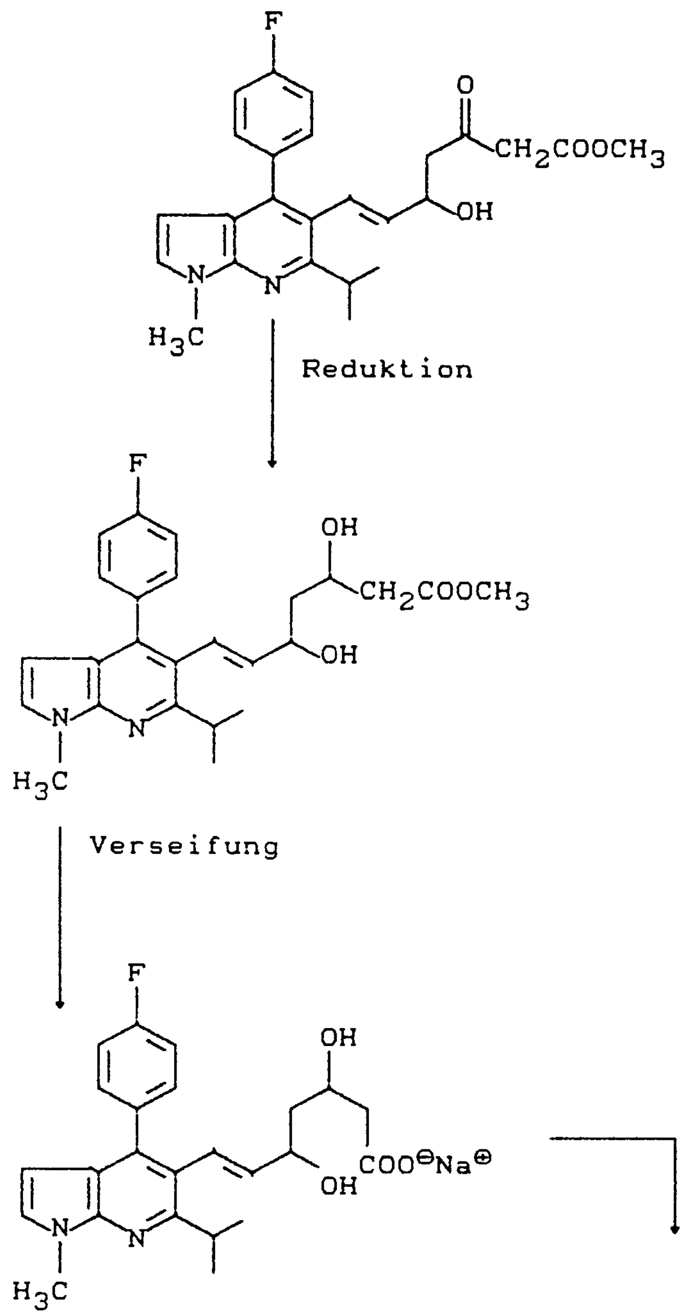
Reduktion
Verseifung

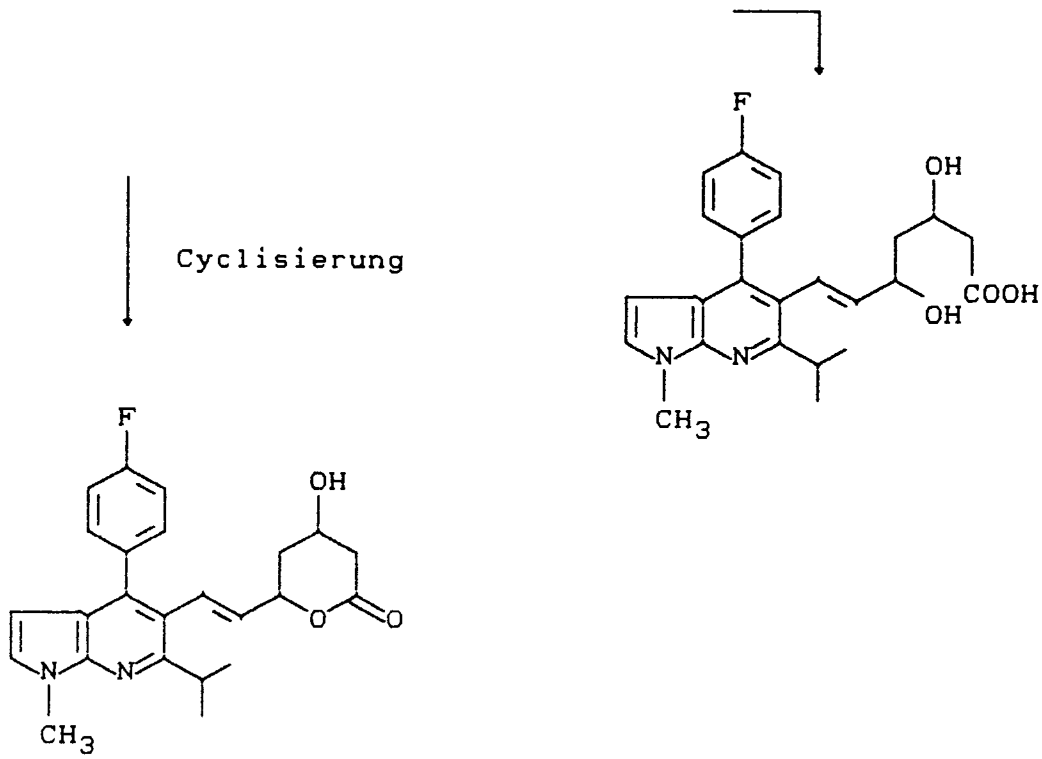


Die Reduktion kann mit den üblichen Reduktionsmitteln, bevorzugt mit solchen, die für die Reduktion von Ketonen zu Hydroxyverbindungen geeignet sind, durchgeführt werden. Besonders geeignet ist hierbei die Reduktion mit Metallhydriden oder komplexen Metallhydriden in inerten Lösemitteln, gegebenenfalls in Anwesenheit eines Trialkylborans. Bevorzugt wird die Reduktion mit komplexen Metallhydriden wie beispielsweise Lithiumboranat, Natriumboranat, Kaliumboranat, Zinkboranat, Lithium-trialkyl-hydrido-boranaten, Natrium-trialkyl-hydrido-boranaten, Natrium-cyano-trihydrido-boranat oder Lithiumaluminiumhydrid durchgeführt. Ganz besonders bevorzugt wird die Reduktion mit Natriumborhydrid, in Anwesenheit von Triethylboran durchgeführt.

Als Lösemittel eignen sich hierbei die üblichen organischen Lösemittel, die sich unter den Reaktionsbedingungen nicht verändern. Hierzu gehören bevorzugt Ether wie beispielsweise Diethylether, Dioxan, Tetrahydrofuran oder Dimethoxyethan, oder Halogenkohlenwasserstoffe wie beispielsweise Dichlormethan, Trichlormethan, Tetrachlormethan, 1,2-Dichlorethan, oder Kohlenwasserstoffe wie beispielsweise Benzol, Toluol oder Xylol. Ebenso ist es möglich Gemische der genannten Lösemittel einzusetzen.

Im Fall, daß E für den Rest der Formel (VIIIa) steht, werden Alkohole wie Methanol, Ethanol oder Propanol, vorzugsweise Ethanol, eingesetzt.

Besonders bevorzugt wird die Reduktion der Ketongruppe zur Hydroxygruppe unter Bedingungen durchgeführt, bei denen die übrigen funktionellen Gruppen wie beispielsweise die Alkoxycarbonylgruppe nicht verändert werden.

Hierzu besonders geeignet ist die Verwendung von Natriumborhydrid als Reduktionsmittel, in Anwesenheit von Triethylboran in inerten Lösemitteln wie vorzugsweise Ethern.

Die Reduktion erfolgt im allgemeinen in einem Temperaturbereich von -80 °C bis +30 °C, bevorzugt von -78 °C bis 0 °C.

Das erfindungsgemäße Verfahren wird im allgemeinen bei Normaldruck durchgeführt. Es ist aber auch möglich, das Verfahren bei Unterdruck oder bei Überdruck durchzuführen (z.B. in einem Bereich von 0,5 bis 5 bar).

Im allgemeinen wird das Reduktionsmittel in einer Menge von 1 bis 2 mol, bevorzugt von 1 bis 1,5 mol bezogen auf 1 mol der Ketoverbindung eingesetzt.

Unter den oben angegebenen Reaktionsbedingungen wird im allgemeinen die Carbonylgruppe zur Hydroxygruppe reduziert, ohne daß Reduktion an der Doppelbindung zur Einfachbindung erfolgt.

Zur Herstellung von Verbindungen der allgemeinen Formel (I), in welchen A für eine Ethylengruppierung steht, kann die Reduktion der Ketone (VIII) unter solchen Bedingungen durchgeführt werden, unter denen sowohl die Carbonylgruppe als auch die Doppelbindung reduziert wird.

Darüberhinaus ist es auch möglich, die Reduktion der Carbonylgruppe und die Reduktion der Doppelbindung in zwei getrennten Schritten durchzuführen.

Die Carbonsäuren im Rahmen der allgemeinen Formel (I) entsprechen der Formel (Ia)

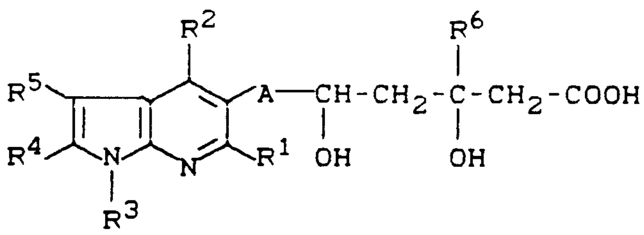

(Ia)

in welcher

$R^1$, $R^2$, $R^3$, $R^4$, $R^5$, $R^6$ und A die oben angegebene Bedeutung haben.

Die Carbonsäureester im Rahmen der allgemeinen Formel (I) entsprechen der Formel (Ib)

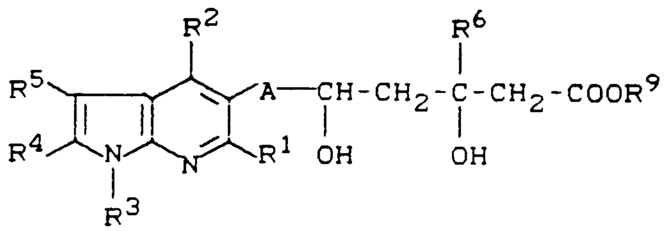

(Ib)

in welcher

$R^1$, $R^2$, $R^3$, $R^4$, $R^5$, $R^6$ und A die oben angegebene Bedeutung haben, und

$R^9$ - für Alkyl steht.

Die Salze der erfindungsgemäßen Verbindungen im Rahmen der allgemeinen Formel (I) entsprechen der Formel (Ic)

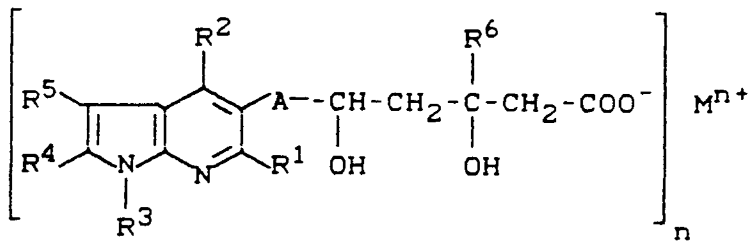

(Ic)

in welcher

$R^1$, $R^2$, $R^3$, $R^4$, $R^5$, $R^6$ und A die oben angegebene Bedeutung haben, und

$M^{n+}$ für ein Kation steht.

Die Lactone im Rahmen der allgemeinen Formel (I) entsprechen der Formel (Id)

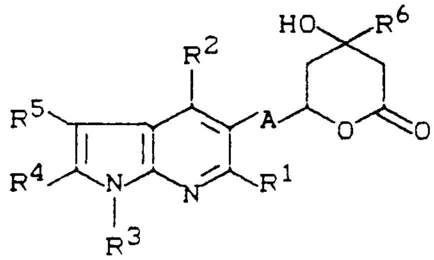

(Id)

in welcher
$R^1$, $R^2$, $R^3$, $R^4$, $R^5$, $R^6$ und A die oben angegebene Bedeutung haben.

Zur Herstellung der erfindungsgemäßen Carbonsäuren der allgemeinen Formel (Ia) werden im allgemeinen die Carbonsäureester der allgemeinen Formel (Ib) oder die Lactone der allgemeinen Formel (d) nach üblichen Methoden verseift. Die Verseifung erfolgt im allgemeinen indem man die Ester oder die Lactone in inerten Lösemitteln mit üblichen Basen behandelt, wobei im allgemeinen zunächst die Salze der allgemeinen Formel (Ic) entstehen, die anschließend in einem zweiten Schritt durch Behandeln mit Säure in die freien Säuren der allgemeinen Formel (Ia) überführt werden können.

Als Basen eignen sich für die Verseifung die üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium- oder Kaliumcarbonat oder Natriumhydrogencarbonat, oder Alkalialkoholate wie Natriumethanolat, Natriummethanolat, Kaliummethanolat, Kaliumethanolat oder Kalium-tert.butanolat. Besonders bevorzugt werden Natriumhydroxid oder Kaliumhydroxid eingesetzt.

Als Lösemittel eignen sich für die Verseifung Wasser oder die für eine Verseifung üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid oder Dimethylsulfoxid. Besonders bevorzugt werden Alkohole wie Methanol, Ethanol, Propanol oder Isopropanol verwendet, Ebenso ist es möglich, Gemische der genannten Lösemittel einzusetzen.

Die Verseifung wird im allgemeinen in einem Temperaturbereich von 0°C bis +100°C, bevorzugt von +20°C bis +80°C durchgeführt.

Im allgemeinen wird die Verseifung bei Normaldruck durchgeführt. Es ist aber auch möglich, bei Unterdruck oder bei Überdruck zu arbeiten (z.B. von 0,5 bis 5 bar).

Bei der Durchführung der Verseifung wird die Base im allgemeinen in einer Menge von 1 bis 3 mol, bevorzugt von 1 bis 1,5 mol bezogen auf 1 mol des Esters bzw. des Lactons eingesetzt. Besonders bevorzugt verwendet man molare Mengen der Reaktanden.

Bei der Durchführung der Reaktion entstehen im ersten Schritt die Salze der erfindungsgemäßen Verbindungen (Ic) als Zwischenprodukte, die isoliert werden können. Die erfindungsgemäßen Säuren (Ia) erhält man durch Behandeln der Salze (Ic) mit üblichen anorganischen Säuren. Hierzu gehören bevorzugt Mineralsäuren wie beispielsweise Chlorwasserstoffsäure, Bromwasserstoffsäure, Schwefelsäure oder Phosphorsäure. Es hat sich bei der Herstellung der Carbonsäuren (Ia) hierbei als vorteilhaft erwiesen, die basische Reaktionsmischung der Verseifung in einem zweiten Schritt ohne Isolierung der Salze anzusäuern. Die Säuren können dann in üblicher Weise isoliert werden.

Zur Herstellung der erfindungsgemäßen Lactone der Formel (Id) werden im allgemeinen die erfindungsgemäßen Carbonsäuren (Ib) nach üblichen Methoden cyclisiert. beispielsweise durch Erhitzen der entsprechenden Säure in inerten organischen Lösemitteln, gegebenenfalls in Anwesenheit von Molsieb.

Als Lösemittel eignen sich hierbei Kohlenwasserstoffe wie Benzol, Toluol, Xylol, Erdölfraktionen, oder Tetralin oder Diglyme oder Triglyme. Bevorzugt werden Benzol, Toluol oder Xylol eingesetzt. Ebenso ist es möglich Gemische der genannten Lösemittel einzusetzen. Besonders bevorzugt verwendet man Kohlenwasserstoffe, insbesondere Toluol, in Anwesenheit von Molsieb.

Die Cyclisierung wird im allgemeinen in einem Temperaturbereich von -40°C bis +200°C, bevorzugt von -25°C bis +50°C durchgeführt.

Die Cyclisierung wird im allgemeinen bei Normaldruck durchgeführt, es ist aber auch möglich, das Verfahren bei Unterdruck oder bei Überdruck durchzuführen (z.B. in einem Bereich von 0,5 bis 5 bar).

Darüberhinaus wird die Cyclisierung auch in inerten organischen Lösemitteln, mit Hilfe von cyclisierenden bzw. wasserabspaltenden Agentien durchgeführt. Als wasserabspaltende Agentien werden hierbei bevorzugt Carbodiimide verwendet. Als Carbodiimide werden bevorzugt N,N'-Dicyclohexylcarbodiimid, N-Cyclohexyl-N'-[2-(N''-methylmorpholinium)ethyl]carbodiimid-paratoluolsulfonat oder N-(3-Dimethylaminopropyl)-N'-ethylcarbodiimid-Hydrochlorid eingesetzt.

Als Lösemittel eignen sich hierbei die üblichen organischen Lösemittel. Hierzu gehören bevorzugt Ether wie Diethylether, Tetrahydrofuran oder Dioxan, oder Chlorkohlenwasserstoffe wie Methylenchlorid, Chloroform oder Tetrachlorkohlenstoff, oder Kohlenwasserstoffe wie Benzol, Toluol, Xylol oder Erdölfraktionen Besonders bevorzugt werden Chlorkohlenwasserstoffe wie beispielsweise Methylenchlorid, Chloroform oder Tetrachlorkohlenstoff, oder Kohlenwasserstoffe wie Benzol, Toluol, Xylol, oder Erdölfraktionen. Besonders bevorzugt werden Chlorkohlenwasserstoffe wie beispielsweise Methylenchlorid, Chloroform oder Tetrachlorkohlenstoff eingesetzt.

Die Reaktion wird im allgemeinen in einem Temperaturbereich von 0°C bis +80°C, bevorzugt von +10°C bis +50°C durchgeführt.

Bei der Durchführung der Cyclisierung hat es sich als vorteilhaft erwiesen, die Cyclisierungsmethode

mit Hilfe von Carbodiimiden als dehydratisierenden Agentien einzusetzen.

Die Trennung der Isomeren in die stereoisomer einheitlichen Bestandteile erfolgt im allgemeinen nach üblichen Methoden wie sie beispielsweise von E.L. Eliel, Stereochemistry of Carbon Compounds, McGraw Hill, 1962 beschrieben werden. Bevorzugt wird hierbei die Trennung der Isomeren auf der Stufe der racemischen Lactone. Besonders bevorzugt wird hierbei die racemische Mischung der trans-Lactone (VII) durch Behandeln entweder mit D-(+)-oder L-(-)-α-Methylbenzylamin nach üblichen Methoden in die diastereomeren Dihydroxyamide (Ie)

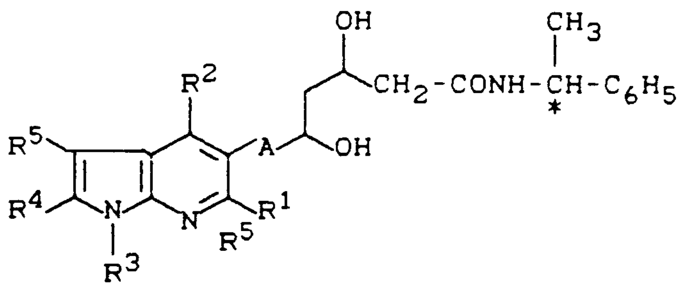

(Ie)

überführt, die anschließend wie üblich durch Chromatographie oder Kristallisation in die einzelnen Diastereomeren getrennt werden können Anschließende Hydrolyse der reinen diastereomeren Amide nach üblichen Methoden, beispielsweise durch Behandeln der diastereomeren Amide mit anorganischen Basen wie Natriumhydroxid oder Kaliumhydroxid in Wasser und/oder organischen Lösemitteln wie Alkoholen z.B. Methanol, Ethanol, Propanol oder Isopropanol, ergeben die entsprechenden enantiomerenreinen Dihydroxysäuren (Ia), die wie oben beschrieben durch Cyclisierung in die enantiomerenreinen Lactone überführt werden können. Im allgemeinen gilt für die Herstellung der erfindungsgemäßen Verbindungen der allgemeinen Formel (I) in enantiomerenreiner Form, daß die Konfiguration der Endprodukte nach der oben beschriebenen Methode abhängig ist von der Konfiguration der Ausgangsstoffe.

Die Isomerentrennung soll im folgenden Schema beispielhaft erläutert werden:

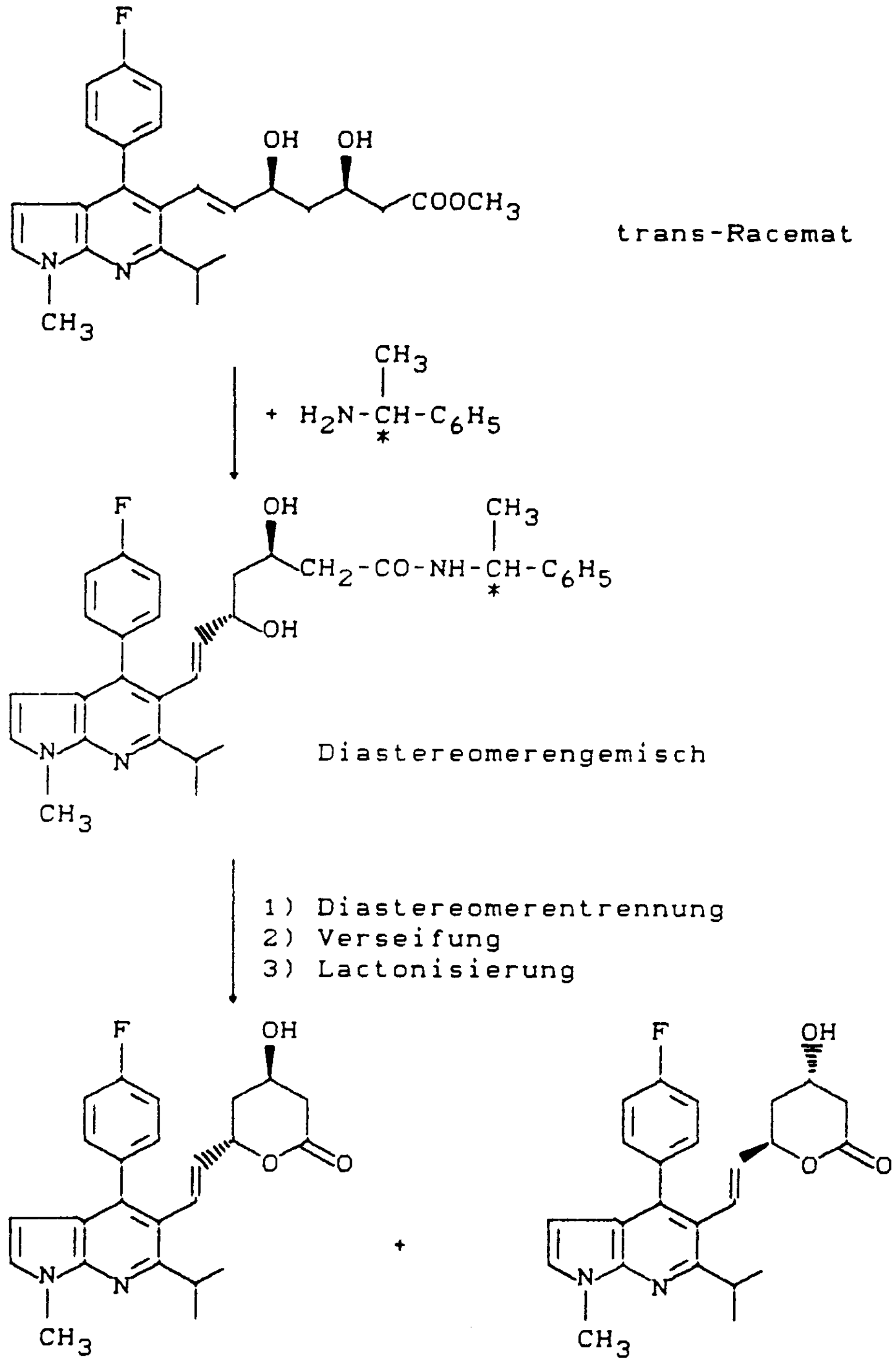


Die als Ausgangsstoffe eingesetzten Ketone der Formel (VIIIa) sind neu und können in Analogie zu den in DE 38 17 298 A1 beschriebenen Verfahren hergestellt.

Die als Ausgangsstoffe eingesetzten Ketone (VIIIb) sind neu.

Es wurde ein Verfahren zur Herstellung der erfindungsgemäßen Ketone der allgemeinen Formel (VIIIb)

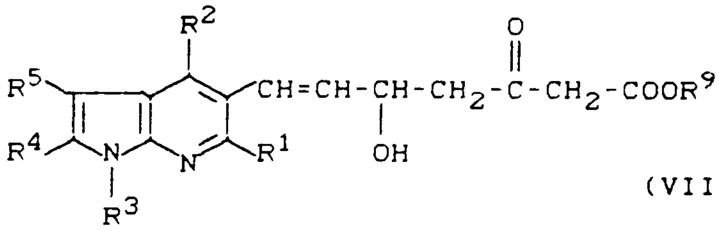

(VIIIb)

in welcher
$R^1$, $R^2$, $R^3$, $R^4$, $R^5$ und $R^9$ die oben angegebene Bedeutung haben,
gefunden, das dadurch gekennzeichnet ist, daß man
Aldehyde der allgemeinen Formel (IX)

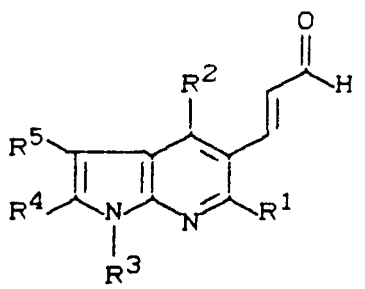

(IX)

in welcher
$R^1$, $R^2$, $R^3$, $R^4$ und $R^5$ die oben angegebene Bedeutung haben,
in inerten Lösemitteln mit Acetessigester der allgemeinen Formel (X)

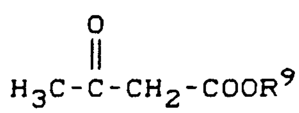

(X)

in welcher
$R^9$ die oben angegebene Bedeutung hat,
in Anwesenheit von Basen umsetzt.

Das erfindungsgemäße Verfahren kann beispielsweise durch folgendes Formelschema erläutert werden:

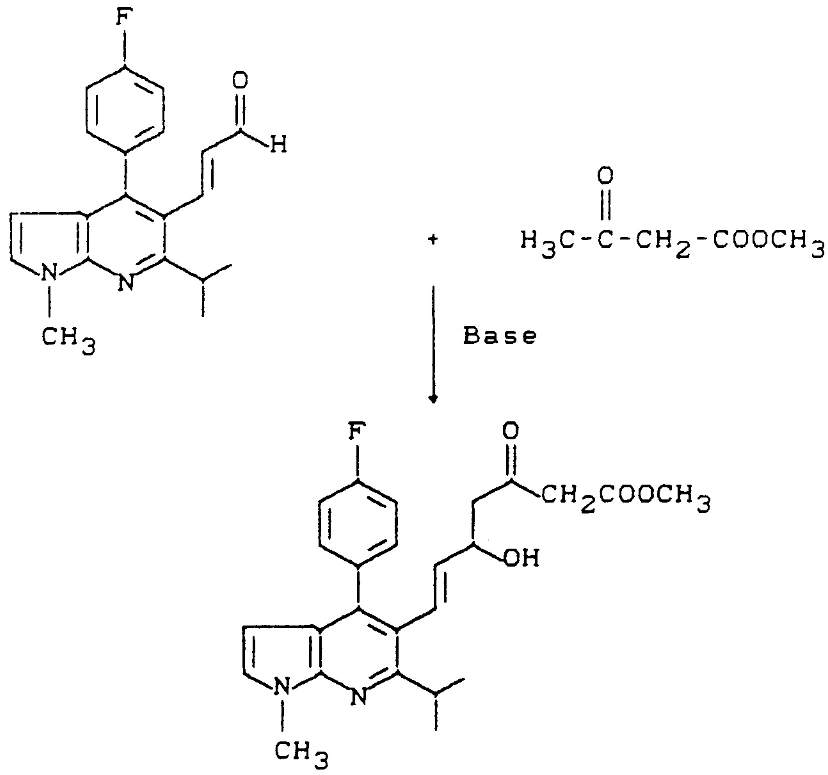


Als Basen kommen hierbei die üblichen stark basischen Verbindungen in Frage. Hierzu gehören bevorzugt lithiumorganische Verbindungen wie beispielsweise N-Butyllithium, sec.Butyllithium, tert.Butyllithium oder Phenyllithium, oder Amide wie beispielsweise Lithiumdiisopropylamid, Natriumamid oder Kaliumamid, oder Lithiumhexamethyldisilylamid, oder Alkalihydride wie Natriumhydrid oder Kaliumhydrid. Ebenso ist es möglich, Gemische der genannten Basen einzusetzen. Besonders bevorzugt werden N-Butyllithium oder Natriumhydrid oder deren Gemisch eingesetzt.

Als Lösemittel eignen sich hierbei die üblichen organischen Lösemittel, die sich unter den Reaktionsbedingungen nicht verändern. Hierzu gehören bevorzugt Ether wie Diethylether, Tetrahydrofuran, Dioxan oder Dimethoxyethan, oder Kohlenwasserstoffe wie Benzol, Toluol, Xylol, Cyclohexan, Hexan oder Erölfraktionen. Ebenso ist es möglich, Gemische der genannten Lösemittel einzusetzen. Besonders bevorzugt werden Ether wie Diethylether oder Tetrahydrofuran verwendet.

Die Reaktion wird im allgemeinen in einem Temperaturbereich von -80° C bis +50° C, bevorzugt von -20° C bis Raumtemperatur durchgeführt.

Das Verfahren wird im allgemeinen bei Normaldruck durchgeführt, es ist aber auch möglich das Verfahren bei Unterdruck oder bei Überdruck durchzuführen, z.B. in einem Bereich von 0,5 bis 5 bar.

Bei der Durchführung des Verfahrens wird der Acetessigester im allgemeinen in einer Menge von 1 bis 2, bevorzugt von 1 bis 1,5 mol, bezogen auf 1 mol des Aldehyds eingesetzt.

Die als Ausgangsstoffe eingesetzten Acetessigester der Formel (X) sind bekannt oder können nach bekannten Methoden hergestellt werden [Beilstein's Handbuch der organischen Chemie III, 632; 438].

Als Acetessigester für das erfindungsgemäße Verfahren seien beispielsweise genannt: Acetessigsäuremethylester, Acetessigsäureethylester, Acetessigsäurepropylester, Acetessigsäureisopropylester.

Die Herstellung der als Ausgangsstoffe eingesetzten Aldehyde der allgemeinen Formel (IX) soll im folgenden beispielhaft für den Fall, daß X für die Gruppe -A-B steht, erläutert werden:

[A]

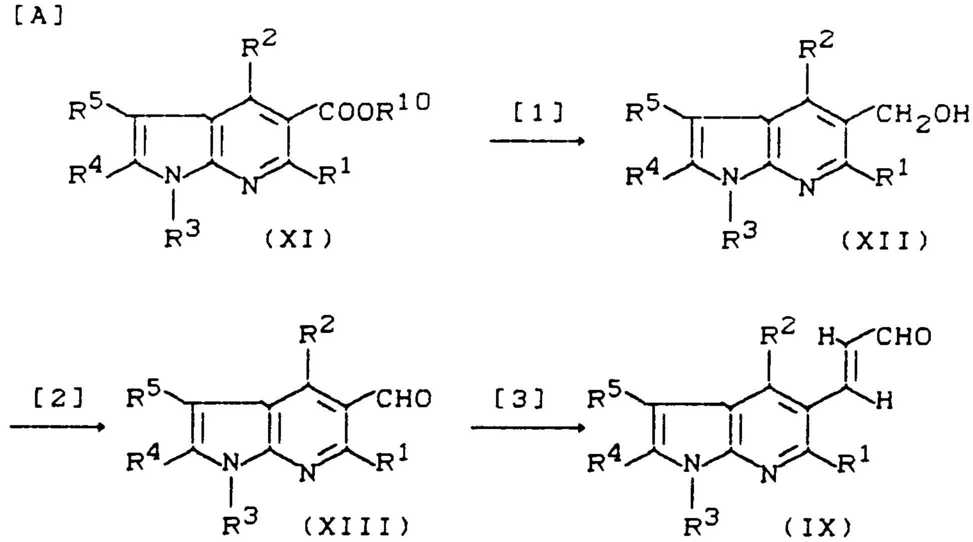


(XI) (XII) (XIII) (IX)

Hierbei werden gemäß Schema A Pyrrolo-pyridine der Formel (XI), in welchen $R^{10}$ für Alkyl mit bis zu 4 Kohlenstoffatomen stehen, im ersten Schritt [1] in inerten Lösemitteln wie Ethern, beispielsweise Diethylether, Tetrahydrofuran oder Dioxan, vorzugsweise Tetrahydrofuran, mit Metallhydriden als Reduktionsmittel, beispielsweise Lithiumaluminiumhydrid, Natriumcyanoborhydrid, Natriumaluminiumhydrid, Diisobutylaluminiumhydrid oder Natrium-bis-(2-methoxyethoxy)-dihydroaluminat, in Temperaturbereichen von -70°C bis +100°C, vorzugsweise von -70°C bis Raumtemperatur, bzw. von Raumtemperatur bis 70°C je nach verwendetem Reduktionsmittel zu den Hydroxymethylverbindungen (XII) reduziert, Vorzugsweise wird die Reduktion mit Diisobutylaluminiumhydrid oder mit Lithiumaluminiumhydrid in Tetrahydrofuran in einem Temperaturbereich von Raumtemperatur bis 80°C durchgeführt. Die Hydroxymethylverbindungen (XII) werden im zweiten Schritt [2] nach üblichen Methoden zu den Aldehyden (XIII) oxidiert, Die Oxidation kann beispielsweise mit Pyridiniumchlorochromat, gegebenenfalls in Anwesenheit von Aluminiumoxid, in inerten Lösemitteln wie Chlorkohlenwasserstoffen, vorzugsweise Methylenchlorid, in einem Temperaturbereich von 0°C bis 60°C, bevorzugt bei Raumtemperatur durchgeführt werden, oder aber mit Trifluoressigsäureanhydrid/Dimethylsulfoxid nach den üblichen Methoden der Swern Oxidation durchgeführt werden, Die Aldehyde (XIII) werden im dritten Schritt [3] mit Diethyl-2-(cyclohexylamino)-vinylphosphonat in Anwesenheit von Natriumhydrid in inerten Lösemitteln wie Ethern, beispielsweise Diethylether, Tetrahydrofuran oder Dioxan, vorzugsweise in Tetrahydrofuran, in einem Temperaturbereich von -20°C bis +40°C, vorzugsweise von -5°C bis Raumtemperatur zu den Aldehyden (IX) umgesetzt.

Die hierbei als Ausgangsstoffe eingesetzten 2H-Pyrrolo-Pyridine (XI) erhält man im allgemeinen gemäß Schema [B], indem man zunächst die Dihydropyridine der Formel (XIV), in welcher $R^{10}$ und $R^{10'}$ gleich oder verschieden sind und die oben angegebene Bedeutung haben, zu den entsprechenden Pyridinen der Formel (XV) oxidiert, dann die Esterfunktion ($COOR^{10'}$) zunächst durch Reduktion in die entsprechenden Hydroxymethylverbindungen (XVI) überführt und anschließend zu entsprechenden Aldehyden (XVII) oxidiert und in einem letzten Schritt nach herkömmlichen Methoden cyclisiert.

[B]

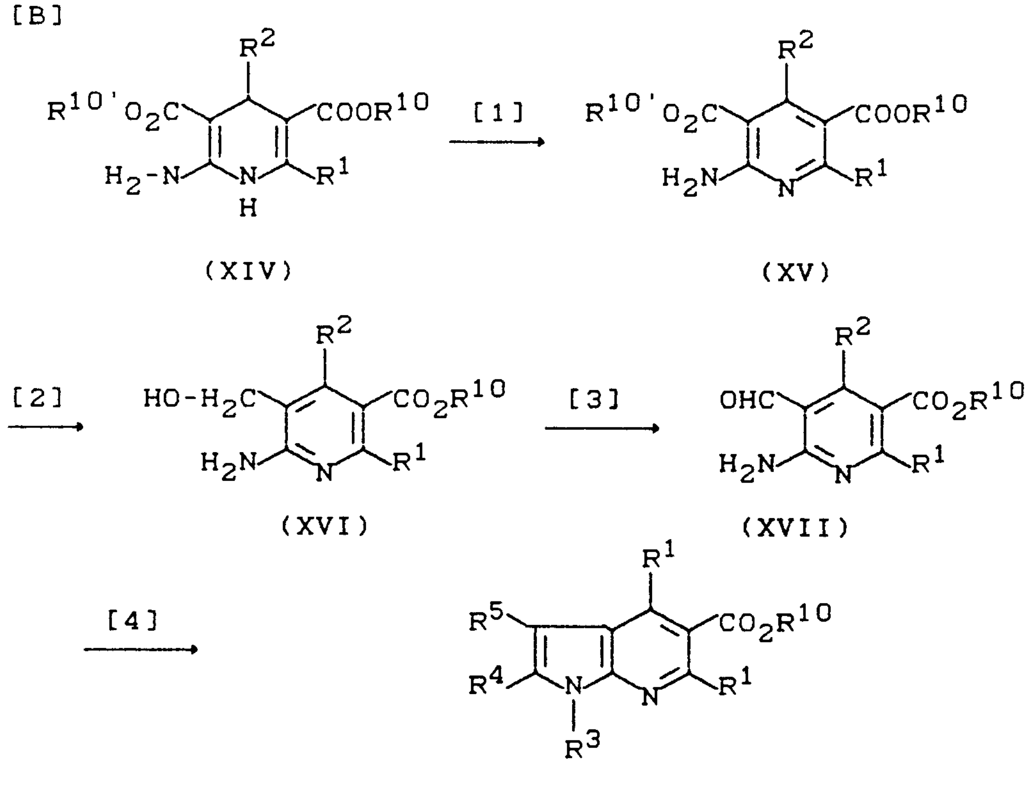


(XIV) (XV) (XVI) (XVII) (XI)

Die hierbei als Ausgangsstoffe eingesetzten Dihydropyridine sind teilweise neu oder bekannt und können nach bekannten Methoden, beispielsweise durch eine Kondensation von Ethoxycarbonyl-acetamidin-hydrochlorid mit (E)-Z-(4-Fluorphenyl)-2-methoxycarbonyl-4-methyl-pent-1-en-3-on hergestellt werden [vgl. außerdem EP-A 88 276, DE-A 28 47 236]. Die Oxidation der Dihydropyridine (XIV) zu den Pyrrolo-Pyridinen (XI) kann beispielsweise mit Chromoxid in Eisessig in einem Temperaturbereich von -20 °C bis +150 °C, vorzugsweise bei Rückflußtemperatur, oder mit 2,3-Dichlor-5,6-dicyan-p-benzochinon als Oxidationsmittel in inerten Lösemitteln wie Chlorkohlenwasserstoffe, vorzugsweise Methylenchlorid in einem Temperaturbereich von 0 °C bis +100 °C, vorzugsweise bei Raumtemperatur durchgeführt werden.

Die Reduktion zu den Hydroxymethylverbindungen der Formel (XVI) [Schritt 2] wird mit geeigneten Reduktionsmitteln, wie beispielsweise Lithiumaluminiumhydrid, Diisobutylaluminiumhydrid oder Natrium-bis-(2-methoxyethoxy)-dihydroaluminat in inerten Lösemitteln, wie beispielsweise Tetrahydrofuran, durchgeführt.

Die Oxidation [Schritt 3] zu den entsprechenden Aldehyden (XVII) erfolgt nach der oben angegebenen Methode.

Die Cyclisierung [Schritt 4] erfolgt durch eine Wittig-Reaktion gegebenenfalls mit substituierten ($R^4/R^5 \neq H$) Phosphonium bzw. Phosphoryliden, beispielsweise mit Methoxymethyltriphenylphosphoniumbromid, in Anwesenheit einer Base in aprotischen Lösemitteln, gefolgt von einer Hydrolyse in wäßriger Säure.

Als Basen eignen sich Alkalihydride und -amide, wie beispielsweise Natriumamid oder Natriumhydrid oder organische Lithiumverbindungen wie beispielsweise Buthyllithium oder Alkoholate wie Kalium-tert.butylat. Bevorzugt ist Natriumamid.

Als Lösemittel eignen sich die üblichen aprotischen organischen Lösemittel wie beispielsweise Tetrahydrofuran, Dimethylformamid oder Dimethylsulfoxid. Bevorzugt ist Tetrahydrofuran.

Die Base wird in einer Menge von 1 bis 4, bevorzugt von 1 bis 2 Moläquivalent eingesetzt.

Als Säuren werden bei der Hydrolyse wäßrige Mineralsäuren, bevorzugt halbkonzentrierte Salzsäure, eingesetzt. Die Hydrolyse erfolgt in einem Temperaturbereich von 0 bis 100 °C, bevorzugt bei 60 bis 100 °C.

Die erfindungsgemäßen Verbindungen der allgemeinen Formel (I) besitzen wertvolle pharmakologische Eigenschaften und können in Arzneimitteln eingesetzt werden. Insbesondere sind sie Inhibitoren der 3-Hydroxy-3-methylglutaryl-Coenzym A (HMG-CoA) Reduktase und infolge dessen Inhibitoren der Cholesterolbiosynthese. Sie können deshalb zur Behandlung von Hyperlipoproteinämie, Lipoproteinämie oder Arteriosklerose eingesetzt werden. Die erfindungsgemäßen Wirkstoffe bewirken außerdem eine Senkung des

Cholesteringehaltes im Blut.

Die Enzymaktivitätsbestimmung wurde modifiziert nach G.C. Ness et al., Archives of Biochemistry and Biophysics 197, 493 - 499 (1979) durchgeführt. Männliche Ricoratten (Körpergewicht 300 - 400 g) wurden 11 Tage mit Altrominpulverfutter, dem 40 g Colestyramin/kg Futter zugesetzt war, behandelt. Nach Dekapitation wurde den Tieren die Leber entnommen und auf Eis gegeben. Die Lebern wurden zerkleinert und im Potter-Elvejem-Homogenisator 3 mal in 3 Volumen 0,1 m Saccharose, 0,05 m KCl, 0,04 m $K_xH_y$ Phosphat, 0,03 m Ethylendiamintetraessigsäure, 0,002 m Dithiothreit (SPE)-Puffer pH 7,2, homogenisiert. Anschließend wurde 15 Minuten bei 15.000 g zentrifugiert und das Sediment verworfen. Der Überstand wurde 75 Minuten bei 100.000 g sedimentiert. Das Pellet wird in 1/4 Volumen SPE-Puffer aufgenommen, nochmals homogenisiert und anschließend erneut 60 Minuten bei 100.000 g zentrifugiert. Das Pellet wird mit der 5-fachen Menge ihres Volumes SPE-Puffer aufgenommen, homogenisiert und bei -78°C eingefroren und gelagert (= Enzymlösung).

Zur Testung wurden die Testverbindungen (oder Mevinolin als Referenzsubstanz) in Dimethylformamid unter Zugabe von 5 Vol.-% 1 n NaOH gelöst und mit 10 µl in verschiedenen Konzentrationen in den Enzymtest eingesetzt. Der Test wurde nach 20 Minuten Vorinkubation der Verbindungen mit dem Enzym bei 37°C gestartet. Der Testansatz betrug 0,380 ml und enthielt 4 µMol Glucose-6-Phosphat, 1,1 mg Rinderserumalbumin, 2,1 µMol Dithiothreit, 0,35 µMol NADP, 1 Einheit Glucose-6-Phosphatdehydrogenase 35 µMol $K_xH_y$-Phosphat PH 7,2, 20 µl Enzympräparation und 56 nMol 3-Hydroxy-3-methyl-glutaryl Coenzym A (Glutaryl-3-$^{14}$C) 100 000 dpm.

Nach Inkubation von 60 Minuten bei 37°C wurde der Ansatz zentrifugiert und 600 µl des Überstandes auf eine 0,7 x 4 cm mit einem 5-Chlorid 100-200 mesh (Anionenaustauscher) gefüllte Säule aufgetragen. Es wurde mit 2 ml dest. Wasser nachgewaschen und Durchlauf plus Waschwasser mit 3 ml Aquasol versetzt und im LKB-Scintillationszähler gezählt. $IC_{50}$-Werte wurden durch Auftrag der prozentualen Hemmung gegen die Konzentration der Verbindung im Test durch Intrapolation bestimmt. Zur Bestimmung der relativen inhibitorischen Potenz wurde der $IC_{50}$-Wert der Referenzsubstanz Mevinolin als 1 gesetzt und mit dem simultan bestimmten $IC_{50}$-Wert der Testverbindung verglichen.

| Bsp.-Nr. | in vitro Relative Aktivität Mevinolin = 1 |
|---|---|
| 1a | 3 |
| 2a | 3 |
| 1b | 3 |
| 2b | 3 |
| 1c | 13 |
| 2c | 3 |
| 1g | 4 |
| 2g | 2 |
| 1f | 17 |
| 2f | 10 |
| 1i | 9 |
| 1h | 33 |
| 2h | 10 |
| 1e | 17 |
| 1d | 27 |
| 1j | 24 |

Zur vorliegenden Erfindung gehören auch pharmazeutische Zubereitungen, die neben inerten, nichttoxischen, pharmazeutisch geeigneten Hilfs- und Trägerstoffen eine oder mehrere Verbindungen der allgemeinen Formel (I) enthalten, oder die aus einem oder mehreren Wirkstoffen der Formel (I) bestehen, sowie Verfahren zur Herstellung dieser Zubereitungen.

Die Wirkstoffe der Formel (I) sollen in diesen Zubereitungen in einer Konzentration von 0,1 bis 99,5 Gew.-%, bevorzugt von 0,5 bis 95 Gew.-% der Gesamtmischung vorhanden sein.

Neben den Wirkstoffen der Formel (I) können die pharmazeutischen Zubereitungen auch andere pharmazeutische Wirkstoffe enthalten.

Die oben aufgeführten pharmazeutischen Zubereitungen können in üblicher Weise nach bekannten Methoden hergestellt werden, beispielsweise mit dem oder den Hilfs- oder Trägerstoffen.

Im allgemeinen hat es sich als vorteilhaft erwiesen, den oder die Wirkstoffe der Formel (I) in

Gesamtmengen von etwa 0,0005 bis etwa 20 mg/kg, bevorzugt in Gesamtmengen von etwa 0,001 mg/kg bis 5 mg/kg Körpergewicht je 24 Stunden, gegebenenfalls in Form mehrerer Einzelgaben, zur Erzielung des gewünschten Ergebnisses zu verabreichen.

Es kann aber gegebenenfalls vorteilhaft sein, von den genannten Mengen abzuweichen, und zwar in Abhängigkeit von der Art und vom Körpergewicht des behandelnden Objekts, vom individuellen Verhalten gegenüber dem Medikament der Art und Schwere der Erkrankung, der Art der Zubereitung und Applikation, sowie dem Zeitpunkt bzw. Intervall, zu welchem die Verabreichung erfolgt.

## Ausgangsverbindungen

### Beispiel I

(E)-Z-1-(4-Fluorphenyl)-2-methoxycarbonyl-4-methyl-pent-1-en-3-on

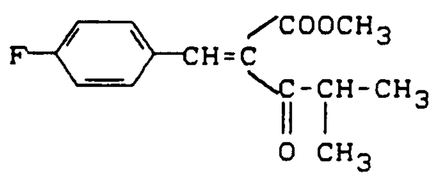

Zu 576,7 g (4 mol) Isobutyrylessigsäuremethylester und 496,5 g (4 mol) 4-Fluorbenzaldehyd in 1 l Isopropanol gibt man eine Lösung von 22,5 ml (0,223 mol) Piperidin und 13,5 ml (0,23 mol) Essigsäure in 100 ml Isopropanol. Man rührt 1 Tag bei Raumtemperatur, engt im Vakuum ein und destilliert den Rückstand im Hochvakuum.

Ausbeute: 840,7 g (84% der Theorie) gelbliches Öl
Kp.: 150-152 ° C (4 mbar)

### Beispiel II

2-Amino-3-ethoxycarbonyl-4-(4-fluorphenyl)-6-isopropyl-5-methoxycarbonyl-1,4-dihydropyridin

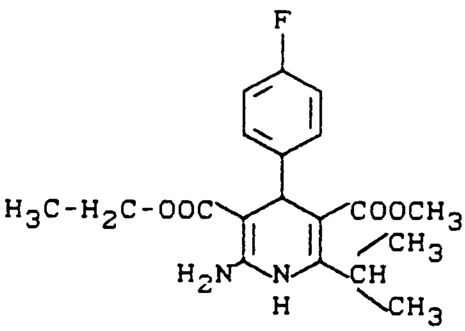

66,6 g (0,4 mol) 3,3-Diaminoacrylsäureethylester Hydrochlorid und 100 g (0,4 mol) der Verbindung aus Beispiel I werden mit 44 ml (0,4 mol) N-Methylmorpholin in 800 ml Isopropanol über Nacht zum Rückfluß gekocht. Man engt im Vakuum ein und chromatographiert den Rückstand in einer Säule (Ø 20 cm) an 2 kg Kieselgel 230-400 mesh mit Petrolether/Essigester (2:1).

Ausbeute: 109,7 g (75,7% der Theorie) farbl. Kristalle
Fp.: 161 ° C (aus Ether/Petrolether)

### Beispiel III

2-Amino-3-ethoxycarbonyl-4-(4-fluorphenyl)-6-isopropyl-5-methoxycarbonyl-pyridin

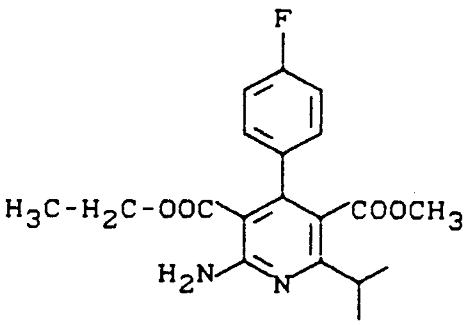

Zu einer Lösung von 36,2 g (0,1 mol) der Verbindung aus Beispiel II in 2 l Dichlormethan gibt man 22,7 g (0,1 mol) 2,3-Dichlor-4,5-dicyano-benzochinon und rührt 40 min bei Raumtemperatur. Man filtiert die Suspension in einer Glasnutsche über 1,5 kg Kieselgel 230 - 400 mesh und eluiert mit einem Gemisch aus Petrolether/Essigester 2:1. Das Eluat wird im Vakuum eingeengt und der verbleibende Rückstand in Ether/Petrolether ausgerührt und abgesaugt.

Ausbeute: 31,6 g (88% der Theorie)
Fp.: 141 °C

Beispiel IV

2-Amino-4-(4-fluorphenyl)-3-hydroxymethyl-6-isopropyl-5-methoxycarbonyl-pyridin

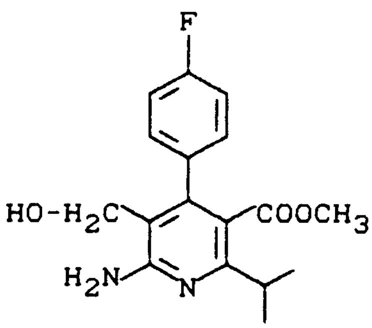

Unter Argon werden 100 ml (0,35 mol) einer 3,5 M Lösung von Natrium-bis-(2-methoxy-ethoxy)-dihydroaluminat in Toluol in 100 ml Tetrahydrofuran p.a. vorgelegt. Man tropft 63 g (175 mmol) der Verbindung aus Beispiel III in 700 ml Tetrahydrofuran gelöst zu und rührt 1 h bei 30 °C nach. Es werden vorsichtig 2 l Wasser zugetropft.

Man trennt die Phasen und wäscht die wässrige Phase 2 mal mit 700 ml Essigester. Die vereinigten organischen Phasen werden mit 500 ml gesättigter Natriumchlorid-Lösung gewaschen und mit Natriumsulfat getrocknet. Man filtriert und engt die Lösung im Vakuum ein. Der Rückstand wird in einer Säule (Ø 6 cm) an 400 g Kieselgel 230 - 400 mesh mit Petrolether/Essigester (1:1) chromatographiert. Man engt das Eluat im Vakuum ein und rührt den Rückstand in Ether/Petrolether aus.

Ausbeute: 45,2 g (81,2% der Theorie) farblose Kristalle
Fp.: 137 °C

Beispiel V

2-Amino-4-(4-fluorphenyl)-3-formyl-5-methoxycarbonyl-6-isopropyl-pyridin

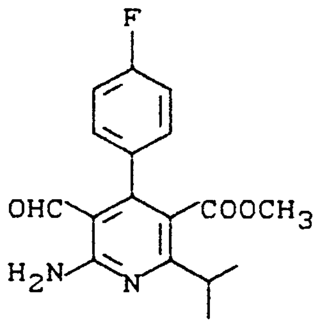

20,7 g (65 mmol) der Verbindung aus Beispiel IV werden in 1,5 l Dichlormethan gelöst, mit 13,3 g (0,13 mol) neutralem Aluminiumoxid und 28 g (0,13 mol) Pyridiniumchlorochromat versetzt und 1 Stunde bei Raumtemperatur gerührt.

Man filtriert die Suspension in einer Glasnutsche über 1 kg Kieselgel 230-400 mesh, eluiert ohne trocken zu saugen mit Petrolether/Essigester 3:1 und engt das Eluat im Vakuum zur Trockene ein.

Ausbeute: 13,4 g (65 % d. Th.)
Fp.: 152 °C

Beispiel VI

4-(4-Fluorphenyl)-6-isopropyl-5-methoxcarbonyl-1H-pyrrolo(2,3-b)pyridin

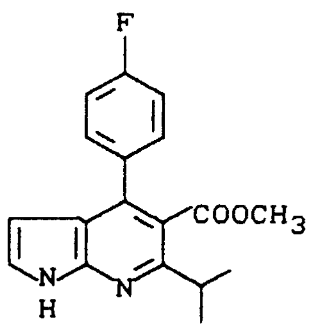

23,9 g (55 mmol) einer Mischung von Methoxymethyltriphenylphosphoniumbromid und Natriumamid werden mit 145 ml wasserfreiem Tetrahydrofuran überschichtet und 15 min kräftig gerührt. Man gibt 17,4 g (55 mmol) der Verbindung aus Beispiel VI zu 145 ml Tetrahydrofuran zu und rührt 1 h bei Raumtemperatur. Es werden weitere 23,9 g (55 mmol) Methoxymethyltriphenylphosphoniumbromid/Natriumamid zugegeben und 60 min bei Raumtemperatur gerührt. Man gibt 185 ml (1,11 mol) 6 M Salzsäure zu, kocht 1 h am Rückfluß und kühlt ab. Es wird mit 5 M Natronlauge neutralisiert und die Phasen getrennt. Man extrahiert die wässrige Phase 2 mal mit Essigester, wäscht die vereinigten organischen Phasen mit gesättigter Natriumchloridlösung, trocknet über Natriumsulfat und engt zur Trockene ein. Der verbleibende Rückstand wird in Dichlormethan gelöst und auf eine Glasfritte (Porosität 3), die 450 g Kieselgel 230-400 mesh enthält, gegeben. Man eluiert ohne Trocken zu saugen mit einem Gradienten aus Petrolether/Essigester 4:1 bis 2:1. Das Eluat wird im Vakuum zur Trockene eingeengt und in einem Gemisch aus Ether/Petrolether ausgerührt.

Ausbeute: 13,8 g (80% der Theorie) farbl. Kristalle
Fp.: 187 °C

Beispiel VII

4-(4-Fluorphenyl)-5-hydroxymethyl-6-isopropyl-1H-pyrrolo(2,3-b)pyridin

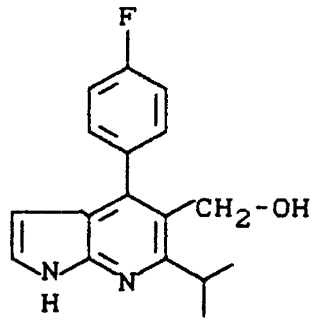

Unter Argon gibt man zu einer Suspension von 15,6 g (50 mmol) der Verbindung aus Beispiel VI in 700 ml Toluol bei -78°C langsam 100 ml einer 1,5 M Lösung von Diisobutylaluminiumhydrid in Toluol, was zu einer klaren Lösung führt. Nach 1 h gibt man bei derselben Temperatur 40 ml 1,5 M Diisobutylaluminiumhydridlösung und rührt eine weitere Stunde. Man erwärmt mit einem Wasserbad auf 0 - 5°C und rührt 1 h bei dieser Temperatur. Es werden vorsichtig 150 ml Wasser und 100 ml Essigester zugegeben, 1 h bei Raumtemperatur gerührt und mit Kieselgur abgesaugt. Man trennt die Phasen, extrahiert die wässrige Phase mit Essigester und wäscht die vereinigten organischen Phasen mit gesättigter Natriumchloridlösung. Es wird über Natriumsulfat getrocknet, im Vakuum zur Trockene eingeengt und der verbleibende Rückstand in Dichlormethan ausgerührt.

Ausbeute: 3,7 g

Der Kieselgurrückstand wird zweimal mit Essigester ausgekocht und abgesaugt. Die Filtrate werden vereinigt, mit gesättigter Natriumchloridlösung gewaschen, über Natriumsulfat getrocknet, im Vakuum eingeengt und in Dichlormethan ausgerührt.

Ausbeute: 8,3 g
Gesamtausbeute: 12,0 g (84% der Theorie)
farblose Kristalle
Fp.: 230°C

Beispiel VIII

4-(4-Fluorphenyl)-5-formyl-6-isopropyl-1H-pyrrolo[2,3-b]pyridin

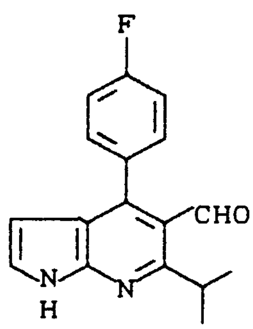

23,9 g (84 mmol) der Verbindung aus Beispiel VII werden in 1,6 l Dichlormethan suspendiert, mit 17,1 g neutralem Aluminiumoxid und 36,1 g (168 mmol) Pyridiniumchlorochromat versetzt und 1 h bei Raumtemperatur gerührt. Man filtriert die Suspension in einer Glasnutsche über 1,2 kg Kieselgel (230-400 mesh) und wäscht ohne trocken zu saugen mit Petrolether/Essigester 3:2 nach. Das Eluat wird im Vakuum zur Trockene eingeengt und der verbleibende Rückstand in Ether ausgerührt.

Ausbeute: 15,4 g (65% der Theorie) farbl. Kristalle
Fp.: 190°C

Beispiel IX-a

(E)-3-[4-(4-Fluorphenyl)-6-isopropyl-1H-pyrrolo[2,3-b]pyridin-5-yl]prop-2-enal

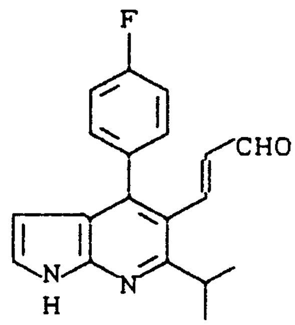

Zu einer Suspension von 2,52 g (84 mmol) 80%igem Natriumhydrid in 60 ml wasserfreiem Tetrahydrofuran tropft man innerhalb von 10 min unter Argon eine Lösung von 11 g (42 mmol) Diethyl-2-(cyclohexylamino)-vinyl-phosphonat in 60 ml Tetrahydrofuran. Am Rückfluß tropft man 9,9 g (35 mmol) der Verbindung aus Beispiel VIII in 100 ml Tetrahydrofuran gelöst zu. Man läßt 90 min am Rückfluß kochen, kühlt ab, versetzt mit Wasser und extrahiert mehrmals mit Essigester. Die vereinigten organischen Phasen werden mit gesättigter Natriumchloridlösung gewaschen und im Vakuum eingeengt. Der Rückstand wird in 300 ml Toluol gelöst und mit 22,9 g (182 mmol) Oxalsäuredihydrat in 350 ml Wasser 1 h unter Rückfluß gekocht. Man kühlt ab, trennt die Phasen und extrahiert die wässrige Phase 2 mal mit Essigester. Die vereinigten organischen Phasen werden mit gesättigter Natriumchloridlösung gewaschen, über Natriumsulfat getrocknet und im Vakuum zur Trockene eingeengt. Den Rückstand löst man in Dichlormethan und filtriert in einer Glasnutsche über 400 g Kieselgel 230 - 400 mesh. Man eluiert ohne trocken zu saugen mit Petrolether/Essigester 1:1, engt das Eluat im Vakuum ein und rührt den Rückstand in Ether aus.

Ausbeute: 9,2 g (85% der Theorie) farbl. Kristalle
Fp.: 236 °C

Beispiel IX-b

(E)-3-[4-(4-Fluorphenyl)-6-isopropyl-1-methyl-1H-pyrrolo[2,3-b]pyridin-5-yl]prop-2-enal

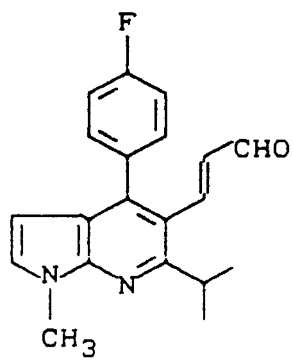

2 g (6,55 mmol) der Verbindung aus Beispiel IX-a werden in 40 ml wasserfreiem Dimethylformamid gelöst, und mit 0,8 g (7,15 mmol) Kalium-tert.butylat versetzt. Man rührt 15 min bei Raumtemperatur, gibt 1,02 g (7,15 mmol) Methyliodid zu und rührt weitere 60 min bei Raumtemperatur. Es wird auf 150 ml Wasser gegossen, mehrmals mit Essigester extrahiert, die vereinigten organischen Phasen mit gesättigter Natriumchloridlösung gewaschen, mit Natriumsulfat getrocknet und im Vakuum zur Trockene eingeengt. Den verbleibenden Rückstand chromatographiert man in einer Säule (Ø 4 cm) an 100 g Kieselgel 230-400 mesh mit Petrolether/Essigester 5:1. Das Eluat wird im Vakuum eingeengt und in Ether/Petrolether ausgerührt.

Ausbeute: 1,15 g (54% der Theorie) farbl. Kristalle
Fp.: 125 °C

In Analogie zu Beispiel IX-b wurden die in Tabelle 1 aufgeführten Verbindungen hergestellt:

Tabelle 1

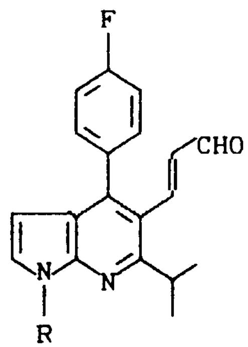

| Bsp.-Nr. | R | Reagenz | Bedingungen | Ausbeute | Fp. |
|---|---|---|---|---|---|
| IX-c | 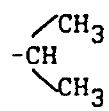 | 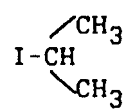 | 2 h, 60° C | 52% | 144° C |
| IX-d | $-CH_2-CN$ | $Cl-CH_2-CN$ | 1 h RT, 1 h 60° C | 19% | amorph. |
| IX-e | $-CH_2-C{\equiv}CH$ | $Br-CH_2-C{\equiv}CH$ 80%ig in Toluol | 1 h RT | 60% | 87° C |
| IX-f | $-CH_2-CH_2-$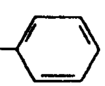 | $Br-CH_2-CH_2-$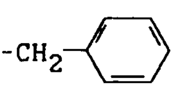 | 1 h RT, + 0,22 äquiv. NaI 1 h RT, 1h 60° C | 31% | 146° C |

Fortsetzung Tabelle 1

| Bsp.-Nr. | R | Reagenz | Bedingungen | Ausbeute | Fp. |
|---|---|---|---|---|---|
| IX-g | $-CH_2-$ 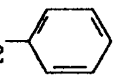 | $Br-CH_2-$ 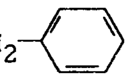 | 1 h RT | 85% | Öl |
| IX-h | $-CH_2-CH=CH_2$ | $Br-CH_2-CH=CH_2$ | 1 h RT | 64% | 85° C |
| IX-i | $-CH_2-$ 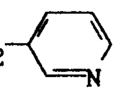 | $Cl-CH_2-$ 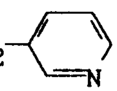 x HCl | x) | 28% | Öl |
| IX-j | $-CH_2-CH_2-CN$ | $CH_2=CH-CN$ | xx) | 17% | 123° C |

x) In Abweichung zur Vorschrift des Beispiels wird die Verbindung IX-i hergestellt, indem man 2,5 Äquivalente Kalium-tert.butylat und 2,5-Äquivalente des aufgeführten Reagenz bei 60° C 2 h rührt.

xx) Ebenfalls in Abweichung zur Vorschrift des Beispiels IX-b wird die Verbindung IX-j hergestellt, indem man 0,1 Äquivalente Kalium-tert.butylat und 2,4 Äquivalente Acrylnitril 3 h bei Raumtemperatur rührt.

Herstellungsbeispiele (allgemeine Formel I)

Beispiel 1a

Methyl-erythro-(E)-7-[4-(4-Fluorphenyl)-6-isopropyl-1H-pyrrolo[2,3-b]pyridin-5-yl]3,5-dihydroxy-hept-6-enoat

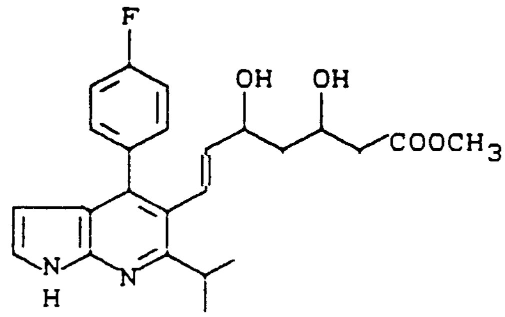

Zu einer Suspension von 0,32 g (10,5 mmol) 80%igem Natriumhydrid in 10 ml wasserfreiem Tetrahydrofuran gibt man bei 0 - 5° C tropfenweise 0,61 g (5,25 mmol) Acetessigsäuremethylester. Nach 15 min tropft man innerhalb von 10 min 6,6 ml (10,85 mmol) 15%iges Butyllithium in Hexan zu und hält weitere 15 min bei 0-5° C Dazu werden 1,1 g(3,5 mmol) der Verbindung aus Beispiel IX-a in 10 ml Tetrahydrofuran zugegeben und 45 min bei Raumtemperatur gerührt. Anschließend versetzt man vorsichtig mit 1,35 g (22,4 mmol) Essigsäure in 25 ml Wasser, extrahiert zweimal mit Essigester, wäscht die vereinigten organischen Phasen mit gesättigter Natriumchloridlösung, trocknet über Natriumsulfat und engt im Vakuum ein. Der verbleibende Rückstand wird in 20 ml Tetrahydrofuran gelöst. Man gibt 4,2 ml (4,2 mmol) einer 1 M Triethylboranlösung in Tetrahydrofuran zu und bläst 5 min Luft durch die Lösung. Bei -78° C gibt man 0,16 g (4,2 mmol) Natriumborhydrid zu. Dann tropft man 3,5 ml Methanol zu und hält 1 h bei -78° C bis -75° C. Man läßt auf Raumtemperatur erwärmen, wobei ab -30° C 11,6 ml 30%iges Wasserstoffperoxid und 30 ml Wasser zugesetzt werden. Es wird zweimal mit Essigester extrahiert, die vereinigten organischen Phasen mit gesättigter Natriumchloridlösung gewaschen, über Natriumsulfat getrocknet und im Vakuum eingeengt. Der verbleibende Rückstand wird in einer Säule (Ø 3 cm) an 40 g Kieselgel 230 - 400 mesh mit Petrolether/Essigester 1:1 chromatographiert. Das Eluat wird im Vakuum eingeengt und in Ether ausgerührt.

Ausbeute: 0,65 g (43% der Theorie)

Fp.: 163° C

In Analogie zu Beispiel 1-a werden die in Tabelle 2 aufgeführten Beispiele aus 7 mmol Natriumhydrid, 5,25 mmol Acetessigester, 10,85 mmol Butyllithium und 3,5 mmol der jeweiligen Verbindung aus Beispiel IX-b-j erhalten wobei mit 19 mmol Essigsäure aufgearbeitet wird.

Tabelle 2

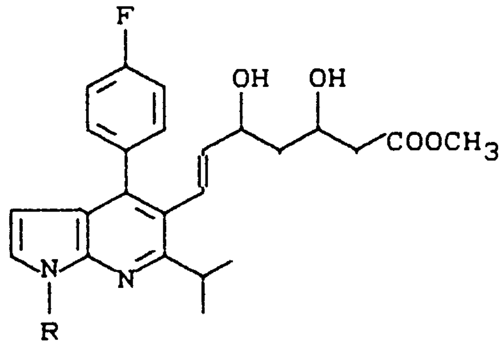

| Bsp. Nr. | R | hergestellt aus Beispiel | Ausbeute (%) | Fp. (°C) |
|---|---|---|---|---|
| 1-b | $-CH_3$ | IX-b | 42 | 74 |
| 1-c | $-CH(CH_3)_2$ | IX-c | 38 | 101 |
| 1-d | $-CH_2-CN$ | IX-d | 3 | amorph. |
| 1-e | $-CH_2-C{\equiv}CH$ | IX-e | 13 | 88 |
| 1-f | $-CH_2-CH_2$ 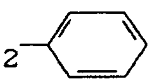 | IX-f | 49 | 124 |
| 1-g | $-CH_2$ 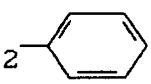 | IX-g | 42 | 126 |
| 1-h | $-CH_2-CH{=}CH_2$ | IX-h | 37 | 104 |
| 1-i | $-CH_2$ 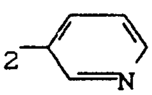 | IX-i | 25 | Öl |
| 1-j | $-CH_2-CH_2-CN$ | IX-j | 18% | Öl |

Beispiel 2-a

Erythro-(E)-7-[4-(4-Fluorphenyl)-6-isopropyl-1H-pyrrolo[2,3-b]pyridin-5-yl]-3,5-dihydroxy-hept-6-en-säure-Natriumsalz

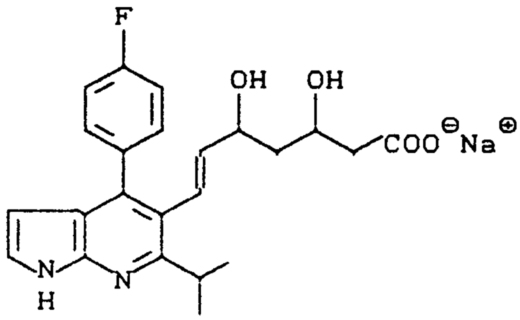

Zu einer Lösung von 141 mg (0,33 mmol) der Verbindung aus Beispiel 1-a in 3,3 ml Tetrahydrofuran gibt man 3,3 ml (0,33 mmol) 0,1 M Natronlauge zu und rührt 1 h bei Raumtemperatur, engt im Vakuum zur Trockene ein und trocknet im Hochvakuum über Phosphorpentoxid.

Ausbeute: 120 mg (83% der Theorie)

Fp.: 243 °C (Zers.)

In Analogie zur Vorschrift des Beispiels 2-a wurden die in der Tabelle 3 aufgeführten Beispiele hergestellt:

Tabelle 3

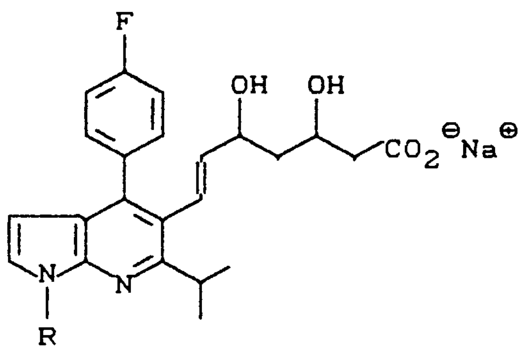

| Bsp-Nr. | R | Ausbeute (% d.Th.) | F(°C) | Ausgangs-verbindung |
|---|---|---|---|---|
| 2-b | $-CH_3$ | 94 | 231 (Zersetz.) | 1-b |
| 2-c | 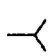 | 49 | 221 (Zersetz.) | 1-c |
| 2-f | $-(CH_2)_2-$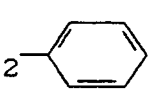 | 91 | 234 (Zersetz.) | 1-f |
| 2-g | $-CH_2-$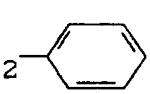 | 99 | 239 (Zersetz.) | 1-g |
| 2-h | $-CH_2-CH=CH_2$ | 89 | 234 (Zersetz.) | 1-h |

Beispiel 3-b

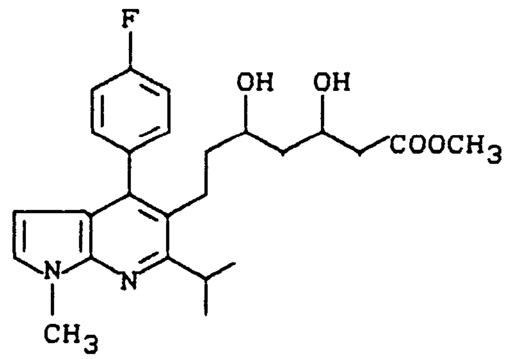

176 mg (0,4 mol) der Verbindung aus Beispiel 2 werden in 30 ml Methanol und 20 µl Triethylamin gelöst und mit 150 mg 10%igem Palladium/Kohle 4,5 h bei Normaldruck und Raumtemperatur hydriert. Man filtriert vom Katalysator ab, engt zur Trockene ein und verteilt den Rückstand zwischen Essigester und gesättigter Kochsalzlösung. Die organische Phase wird getrocknet, eingeengt und aus Ether/Petrolether kristallisiert.

Ausbeute: 91 mg (51% der Theorie) farbl. Kristalle
Fp.: 97°C

## Patentansprüche

**1.** Substituierte Pyrrolo-pyridine der allgemeinen Formel (I)

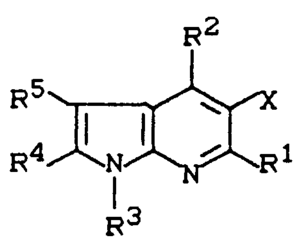

(I)

in welcher

$R^1$ - für geradkettiges oder verzweigtes Alkyl mit bis zu 8 Kohlenstoffatomen steht,
- für Cycloalkyl mit 3 bis 6 Kohlenstoffatomen steht,

$R^2$ - für Phenyl steht, das gegebenenfalls bis zu 2-fach gleich oder verschieden durch geradkettiges oder verzweigtes Alkyl mit bis zu 6 Kohlenstoffatomen, Trifluormethyl, Hydroxymethyl, Phenoxy, Benzyl, Benzyloxy oder Halogen substituiert ist,

$R^3$ - für Wasserstoff oder
- für geradkettiges oder verzweigtes Alkyl mit bis zu 10 Kohlenstoffatomen steht, das gegebenenfalls durch Cyano, geradkettiges oder verzweigtes Alkoxy mit bis zu 6 Kohlenstoffatomen, Halogen, Pyridyl, Chinolinyl, Furyl, Thienyl, Naphthyl oder Phenyl substituiert ist, das seinerseits durch Halogen, Cyano, Nitro, geradkettiges oder verzweigtes Alkyl oder Alkoxy mit jeweils bis zu 6 Kohlenstoffatomen substituiert sein kann,
- für geradkettiges oder verzweigtes Alkenyl oder Alkinyl mit jeweils bis zu 8 Kohlenstoffatomen steht, oder

- für Cycloalkyl mit 3 bis 6 Kohlenstoffatomen oder Phenyl steht,

$R^4$ und $R^5$ gleich oder verschieden sind und
- für Wasserstoff oder
- für geradkettiges oder verzweigtes Alkyl mit bis zu 6 Kohlenstoffatomen stehen, das gegebenenfalls durch Hydroxy, Halogen, Cyano oder Alkoxy mit bis zu 4 Kohlenstoffatomen substituiert ist,

X - für einen Rest der Formel -A-B steht,

worin

A - eine Gruppe der Formel $-CH_2-CH_2-$ oder -CH = CH-bedeutet,

B - eine Gruppe der Formel

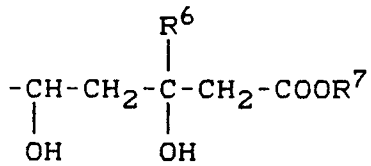 oder 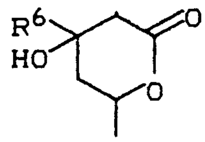

bedeutet

worin

$R^6$ - Wasserstoff oder geradkettiges oder verzweigtes Alkyl mit bis zu 10 Kohlenstoffatomen bedeutet

und

$R^7$ - Wasserstoff oder geradkettiges oder verzweigtes Alkyl mit bis zu 10 Kohlenstoffatomen bedeutet, das durch Phenyl substituiert sein kann, oder
- Aryl mit 6 bis 10 Kohlenstoffatomen oder ein Kation bedeutet,

oder

- für einen Rest der Formel

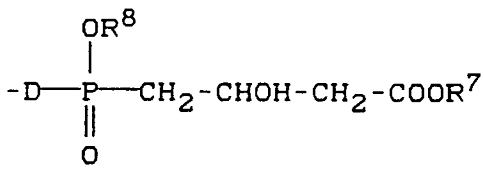

steht,

worin

D - einen Rest der Formel $-(CH_2)t$, -CH = CH-, -C≡C- oder $-CH_2-O-$ bedeutet, worin letzterer über O an das Phosphoratom gebunden ist,

$R^7$ die oben angegebene Bedeutung von $R^7$ hat

$R^8$ - Wasserstoff oder geradkettiges oder verzweigtes Alkyl mit bis zu 4 Kohlenstoffatomen bedeutet,

t - die Zahl 1 oder 2 bedeutet,

und deren Salze.

**2.** Substituierte Pyrrolo-pyridine der allgemeinen Formel nach Anspruch 1

in welcher

$R^1$ - für geradkettiges oder verzweigtes Alkyl mit bis zu 6 Kohlenstoffatomen steht,
- für Cyclopropyl, Cyclopentyl oder Cyclohexyl steht,

$R^2$ - für Phenyl steht, das gegebenenfalls durch geradkettiges oder verzweigtes Alkyl mit bis zu 4 Kohlenstoffatomen, Trifluormethyl, Fluor, Chlor oder Brom substituiert ist,

$R^3$ - für Wasserstoff oder
- für geradkettiges oder verzweigtes Alkyl mit bis zu 8 Kohlenstoffatomen steht, das gegebenenfalls durch Cyano, geradkettiges oder verzweigtes Alkoxy mit bis zu 4 Kohlenstoffatomen, Fluor, Chlor, Brom, Pyridyl oder Phenyl substituiert ist, das seinerseits durch Fluor, Chlor, Brom, Cyano, Nitro, geradkettiges oder verzweigtes Alkyl oder Alkoxy mit jeweils bis zu 4 Kohlenstoffatomen substituiert sein kann,

- für geradkettiges oder verzweigtes Alkenyl oder Alkinyl mit jeweils bis zu 6 Kohlenstoffatomen steht,
- für Cyclopropyl, Cyclopentyl, Cyclohexyl oder Phenyl steht,

$R^4$ und $R^5$ für Wasserstoff stehen,

X - für einen Rest der Formel -A-B steht,

worin

A - eine Gruppe der Formel $-CH_2-CH_2-$ oder -CH = CH- bedeutet,

B - eine Gruppe der Formel

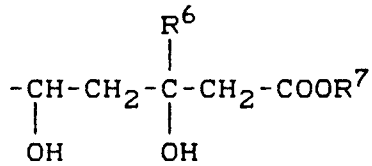 oder 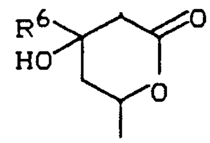

bedeutet,
worin

$R^6$ - Wasserstoff bedeutet

und

$R^7$ - Wasserstoff oder geradkettiges oder verzweigtes Alkyl mit bis zu 8 Kohlenstoffatomen oder Benzyl bedeutet, oder
- Phenyl oder ein Kation bedeutet

und deren Salze,

**3.** Substituierte Pyrrolo-pyridine der allgemeinen Formel nach Anspruch 1

in welcher

$R^1$ - für geradkettiges oder verzweigtes Alkyl mit bis zu 4 Kohlenstoffatomen oder Cyclopropyl steht,

$R^2$ - für Phenyl steht, das gegebenenfalls durch Methyl, Trifluormethyl, Fluor oder Chlor substituiert ist,

$R^3$ - für Wasserstoff oder
- für geradkettiges oder verzweigtes Alkyl mit bis zu 6 Kohlenstoffatomen steht, das gegebenenfalls durch Cyano, Methoxy, Fluor, Pyridyl oder Phenyl substituiert ist,
- für geradkettiges oder verzweigtes Alkenyl oder Alkinyl mit jeweils bis zu 4 Kohlenstoffatomen steht,
- für Cyclopentyl oder Cyclohexyl steht,

$R^4$ und $R^5$ für Wasserstoff stehen,

X - für einen Rest der Formel -A-B steht,

worin

A - eine Gruppe $-CH_2-CH_2-$ oder -CH = CH- bedeutet,

B - eine Gruppe der Formel

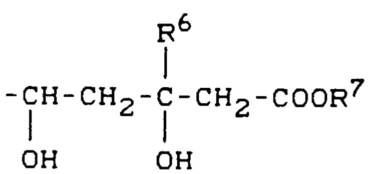 oder 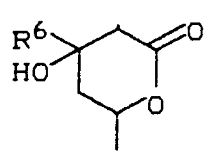

bedeutet,
worin

$R^6$ - Wasserstoff bedeutet

und

$R^7$ - Wasserstoff, Methyl, Ethyl, Propyl, Isopropyl, Butyl, Isobutyl, tert.-Butyl oder Benzyl bedeutet, oder
- ein Natrium-, Kalium-, Calcium-, Magnesium- oder Ammoniumion bedeutet

und deren Salze,

**4.** Verfahren zur Herstellung von substituierten Pyrrolo-pyridinen der allgemeinen Formel

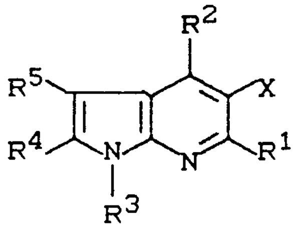

(I)

in welcher

$R^1$ - für geradkettiges oder verzweigtes Alkyl mit bis zu 8 Kohlenstofatomen steht,
- für Cycloalkyl mit 3 bis 6 Kohlenstoffatomen steht,

$R^2$ - für Phenyl steht, das gegebenenfalls bis zu 2-fach gleich oder verschieden durch geradkettiges oder verzweigtes Alkyl mit bis zu 6 Kohlenstoffatomen, Trifluormethyl, Hydroxymethyl, Phenoxy, Benzyl, Benzyloxy oder Halogen substituiert ist,

$R^3$ - für Wasserstoff oder
- für geradkettiges oder verzweigtes Alkyl mit bis zu 10 Kohlenstoffatomen steht, das gegebenenfalls durch Cyano, geradkettiges oder verzweigtes Alkoxy mit bis zu 6 Kohlenstoffatomen, Halogen, Pyridyl, Chinolinyl, Furyl, Thienyl, Naphthyl oder Phenyl substituiert ist, das seinerseits durch Halogen, Cyano, Nitro, geradkettiges oder verzweigtes Alkyl oder Alkoxy mit jeweils bis zu 6 Kohlenstoffatomen substituiert sein kann,
- für geradkettiges oder verzweigtes Alkenyl oder Alkinyl mit jeweils bis zu 8 Kohlenstoffatomen steht, oder

- für Cycloalkyl mit 3 bis 6 Kohlenstoffatomen oder Phenyl steht,

$R^4$ und $R^5$ gleich oder verschieden sind und
- für Wasserstoff oder
- für geradkettiges oder verzweigtes Alkyl mit bis zu 6 Kohlenstoffatomen stehen, das gegebenenfalls durch Hydroxy, Halogen, Cyano oder Alkoxy mit bis zu 4 Kohlenstoffatomen substituiert ist,

X - für einen Rest der Formel -A-B steht,

worin

A - eine Gruppe der Formel $-CH_2-CH_2-$ oder -CH=CH-bedeutet,

B - eine Gruppe der Formel

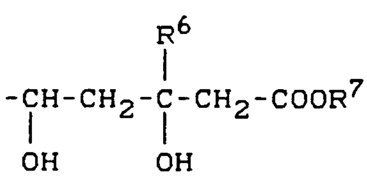

oder

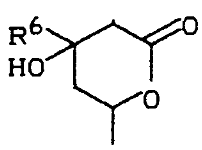

bedeutet

worin

$R^6$ - Wasserstoff oder geradkettiges oder verzweigtes Alkyl mit bis zu 10 Kohlenstoffatomen bedeutet

und

$R^7$ - Wasserstoff oder geradkettiges oder verzweigtes Alkyl mit bis zu 10 Kohlenstoffatomen bedeutet, das durch Phenyl substituiert sein kann, oder
- Aryl mit 6 bis 10 Kohlenstoffatomen oder ein Kation bedeutet,

oder

- für einen Rest der Formel

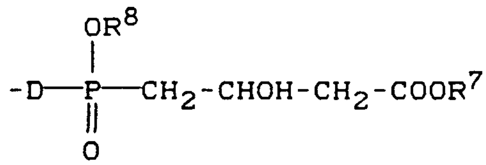

steht,
worin

D - einen Rest der Formel $-(CH_2)t$, $-CH=CH-$, $-C\equiv C-$ oder $-CH_2-O-$ bedeutet, worin letzterer über O an das Phosphoratom gebunden ist,

$R^7$ die oben angegebene Bedeutung von $R^7$ hat

$R^8$ - Wasserstoff oder geradkettiges oder verzweigtes Alkyl mit bis zu 4 Kohlenstoffatomen bedeutet,

t - die Zahl 1 oder 2 bedeutet,

dadurch gekennzeichnet, daß man Ketone der allgemeinen Formel

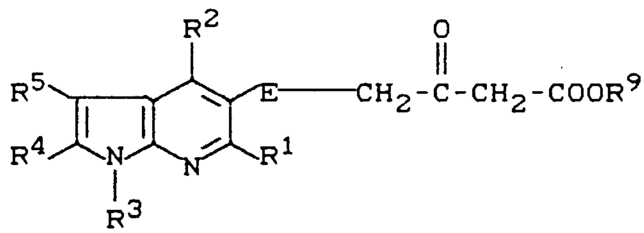
(VIII)

in welcher

$R^1$, $R^2$, $R^3$, $R^4$ und $R^5$ die oben angegebene Bedeutung haben,

$R^9$ - für Alkyl steht,

und

E für eine Gruppe

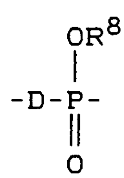
(VIIIa)

oder

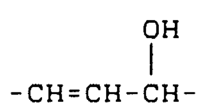
(VIIIb)

steht

reduziert,

im Fall der Herstellung der Säuren die Ester verseift,

im Fall der Herstellung der Lactone die Carbonsäuren cyclisiert,

im Fall der Herstellung der Ethylenverbindungen (A = $-CH_2-CH_2-$) die Ethenylverbindungen (A = $-CH=CH-$) nach üblichen Methoden hydriert,

und gegebenenfalls Isomeren trennt.

**5.** Substituierte Pyrrolo-pyridine nach den Ansprüchen 1 bis 3 zur Bekämpfung von Krankheiten.

**6.** Arzneimittel enthaltend mindestens ein substituiertes Pyrrolo-pyridin nach den Ansprüchen 1 bis 3.

**7.** Verfahren zur Herstellung eines Arzneimittels nach Anspruch 6, dadurch gekennzeichnet, daß man die substituierten Pyrrolo-pyridine mit Hilfe von üblichen Hilfs- und Trägerstoffen in eine geeignete Applikationsform bringt.

**8.** Verwendung von substituierten Pyrrolo-pyridinen nach den Ansprüchen 1 bis 3 zur Herstellung von Arzneimitteln.

**9.** Verwendung von substituierten Pyrrolo-pyridinen nach den Ansprüchen 1 bis 3 zur Herstellung von Arzneimitteln zur Behandlung von Hyperlipoproteinämie, Lipoproteinämie und Arteriosklerose.

**10.** Verwendung von substituierten Pyrrolo-pyridinen nach den Ansprüchen 1 bis 3 zur Behandlung von Krankheiten.

Europäisches Patentamt

**EUROPÄISCHER TEILRECHERCHENBERICHT,** der nach Regel 45 des Europäischen Patentübereinkommens für das weitere Verfahren als europäischer Recherchenbericht gilt

Nummer der Anmeldung
EP 91110832.2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | US - A - 4 939 159 (ANDERSON) * Zusammenfassung; Spalte 4, Reaktionsschema A; Spalte 11, Reaktionsschema G; Spalte 3, Zeilen 59,60 * -- | 1,4-9 | C 07 D 471/04<br>C 07 F 9/6561<br>C 07 F 9/32<br>A 61 K 31/435<br>//(C 07 D 471/04<br>C 07 D 221:00<br>C 07 D 209:00) |
| A | DE - A - 1 595 949 (MERCK) ---- | | |

RECHERCHIERTE SACHGEBIETE (Int. Cl.5)
C 07 D 471/00
C 07 F 9/00

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-9
Unvollständig recherchierte Patentansprüche: –
Nicht recherchierte Patentansprüche: 10
Grund für die Beschränkung der Recherche:

Artikel 52(4) EPÜ; Verfahren zur therapeutischen Behandlung des menschlichen oder tierischen Körpers

| Recherchenort | Abschlußdatum der Recherche | Prüfer |
|---|---|---|
| WIEN | 08-08-1991 | ONDER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPA Form 1505.1 03.82